(19) Europäisches Patentamt
European Patent Office
Office européen des brevets

(11) Veröffentlichungsnummer: **0 006 532**
**A1**

(12) **EUROPÄISCHE PATENTANMELDUNG**

(21) Anmeldenummer: **79101909.4**

(22) Anmeldetag: **12.06.79**

(51) Int. Cl.³: **C 07 D 499/64**, C 07 D 501/36, C 07 D 501/32, C 07 D 501/22, A 61 K 31/43, A 61 K 31/545

(30) Priorität: **28.06.78 DE 2828261**

(43) Veröffentlichungstag der Anmeldung: **09.01.80 Patentblatt 80/1**

(84) Benannte Vertragsstaaten: **AT BE CH DE FR GB IT LU NL SE**

(71) Anmelder: **Dr. Karl Thomae GmbH, Postfach 720, D-7950 Biberach (Riss) (DE)**

(72) Erfinder: **Wetzel, Bernd, Dr. Dipl.-Chem., Kapellenweg 21, D-7950 Biberach 1 (DE)**
Erfinder: **Woitun, Eberhard, Dr. Dipl.-Chem., Stecherweg 17, D-7950 Biberach 1 (DE)**
Erfinder: **Maier, Roland, Dr. Dipl.-Chem., Bodelschwinghstrasse 39, D-7950 Biberach 1 (DE)**
Erfinder: **Reuter, Wolfgang, Dr. Dipl.-Chem., Nelkenweg 10, D-7951 Lauperthausen (DE)**
Erfinder: **Lechner, Uwe, Dr., Panoramastrasse 12, D-7951 Ummendorf (DE)**
Erfinder: **Goeth, Hanns, Dr., Oberer Bühl 6, D-7950 Biberach 1 (DE)**

(54) **Neue Lactame, Verfahren zu ihrer Herstellung und diese Verbindungen enthaltende Arzneimittel.**

(57) Die Erfindung betrifft neue β-Lactame der allgemeinen Formel

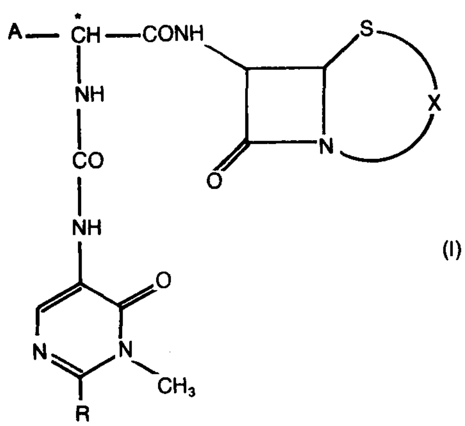

(I)

und gegebenenfalls deren physiologisch verträglichen Salze, zwei Verfahren zu deren Herstellung und diese Verbindungen enthaltende Arzneimittel.

Es handelt sich, je nach der Bedeutung von X um Penicillin- oder Cephalosporinderivate, wobei A einen gegebenenfalls substituierten Phenylrest, einen Thienyl-, Furyl-, Cyclohexyl-, Cyclohexenyl- oder Cyclohexadienylrest darstellt und R ein Wasserstoffatom, verschiedene aliphatische oder aromatische Kohlenwasserstoffreste, Aminogruppen, substituierte Aminocarboxy- oder Aminosulfonylgruppen oder Morpholino- oder in 4-Stellung substituierte Piperazinoreste, Alkylsulfinyl- oder Alkylsulfonylreste oder gegebenenfalls substituierte Mercaptogruppen bedeuten. Die Verbindungen der allgemeinen Formel I zeigen in vitro und in vivo eine starke Wirkung gegen grampositive und gramnegative Bakterien und erlauben bei oraler und parenteraler Gabe die Erlangung hoher Spiegel im Gewebe, Serum, in Organen und im Urin.

EP 0 006 532 A1

0006532

Case 5/738
Dr.Bu/ma
Auslandstext

DR. KARL THOMAE GMBH, BIBERACH AN DER RISS
==========================================

Neue Lactame, Verfahren zu ihrer Herstellung und diese Verbindungen enthaltende Arzneimittel

Die Erfindung betriffft neue ß-Lactame der allgemeinen Formel I

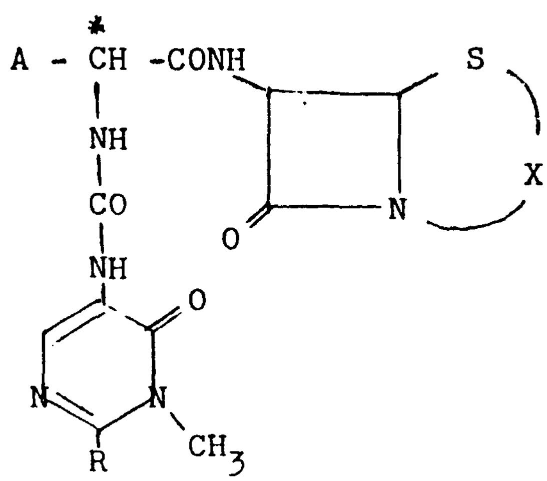

, (I)

gegebenenfalls ihre physiologisch verträgliche Salze mit anorganischen oder organischen Basen, Verfahren zur Herstellung der Verbindungen und diese Verbindungen enthaltende Arzneimittel.

In der obigen allgemeinen Formel I bedeutet

A die Phenyl-, 4-Hydroxyphenyl-, 2- oder 3-Thienyl-, 2- oder 3-Furyl-, Cyclohexyl-, Cyclohexen-1-yl-, Cyclohexa-1,4-dien-1-yl-gruppe sowie einen in 3,4-Stellung disubstituierten Phenylrest, wobei die Substituenten dieses Restes gleich oder voneinander verschieden und Chloratome, Hydroxy- oder Methoxygruppen sein können,

R ein Wasserstoffatom, einen aliphatischen verzweigten oder unverzweigten gesättigten oder ungesättigten Kohlenwasserstoffrest mit 1 bis 4 Kohlenstoffatomen, die Phenyl- oder Benzylgruppe, eine Cycloalkylgruppe mit 3 bis 7 Kohlenstoffatomen, die Hydroxygruppe, eine Alkoxygruppe mit 1 bis 4 Kohlenstoffatomen, eine Alkylsulfinyl oder Alkylsulfonylgruppe mit 1 bis 4 Kohlenstoffatomen, die Mercaptogruppe, eine Alkylmercaptogruppe mit 1 bis 4 Kohlenstoffatomen, die Aminogruppe, eine Alkyl- oder Dialkylaminogruppe mit 1 bis 8 Kohlenstoffatomen im Alkylteil, eine durch eine Hydroxygruppe substituierte Alkylaminogruppe mit 1 bis 4 Kohlenstoffatomen, eine Cycloalkylaminogruppe mit 3 bis 7 Kohlenstoffatomen, die 4-Hydroxycyclohexylaminogruppe, die Benzylaminogruppe, die Anilinogruppe, eine Morpholino-, N-Formylpiperazino- oder N-Acetylpiperazinogruppe oder eine Gruppe der allgemeinen Formel

$$-NHCOR_1,$$

in der $R_1$ eine Alkyl- oder Alkenylgruppe mit 1 bis 6 Kohlenstoffatomen, eine Cycloalkylgruppe mit 3 bis 7 Kohlenstoffatomen, die Benzylgruppe, die Phenylgruppe, eine Wasserstoffatom, die Trifluormethylgruppe, eine Alkoxygruppe mit 1 bis 6 Kohlenstoffatomen, die Benzyloxygruppe, eine Cycloalkoxygruppe mit 3 bis 7 Kohlenstoffatomen, die Aminogruppe, eine Alkyl- oder Dialkylaminogruppe mit 1 bis 8 Kohlenstoffatomen im Alkylteil, eine Cycloalkylaminogruppe mit 3 bis 6 Kohlenstoffatomen oder die Benzylaminogruppe darstellt; oder R ist eine Gruppe der allgemeinen Formel

$$-NHSO_2R_2,$$

in der $R_2$ eine Alkylgruppe mit 1 bis 6 Kohlenstoffatomen, eine Cycloalkylgruppe mit 3 bis 7 Kohlenstoffatomen, die Benzylgruppe, die Phenyl- oder Toluylgruppe bedeutet; oder eine Gruppe der Formel

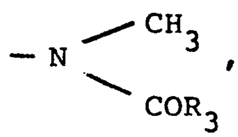

in der $R_3$ ein Wasserstoffatom oder eine Alkylgruppe mit 1 bis 3 Kohlenstoffatomen bedeutet,.

X die Gruppen:

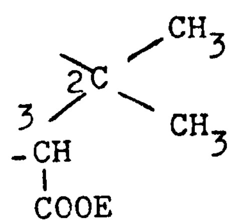

und

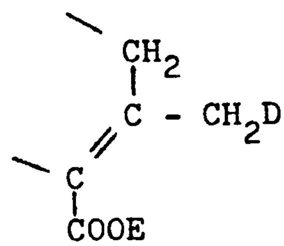

worin D ein Wasserstoffatom, die Acetoxy- oder Carbamoyloxygruppe oder die Gruppe -SHet darstellt, wobei

Het die 3-Methyl-1,2,4-thiadiazol-5-yl-, 5-Methyl-1,3,4-oxadiazol-2-yl-, 1,3,4-Thiadiazol-2-yl-, 5-Methyl-1,3,4-thiadiazol-2-yl-, Tetrazol-5-yl-, 1-Methyl-tetrazol-5-yl-, 1,2,3-Triazol-4-yl- oder 4-Methyl-oxazol-2-ylgruppe bedeutet, und

E ein Wasserstoffatom oder eine in vitro oder in vivo leicht spaltbare Schutzgruppe darstellt. Als Carboxylschutzgruppen kommen im Sinne der Erfindung solche in Frage, welche auch bisher schon auf dem Gebiet der Penicilline und Cephalosporine eingesetzt wurden, insbesondere esterbildende Gruppen, die durch Hydrogenolyse oder Hydrolyse oder andere Behandlungen unter milden Bedingungen entfernt werden können oder esterbildende Gruppen, welche leicht im lebenden Organismus abgespalten werden können. Beispiele für in vitro leicht spaltbare Schutzgruppen sind z. B. die Benzyl-, Diphenylmethyl-, Trityl-, t-Butyl-, die 2,2,2-Trichloräthyl- oder die Trimethylsilylgruppe.
Beispiele für in vivo leicht spaltbare Schutzgruppen sind z. B. Alkanoyloxyalkylgruppen, wie z. B. die Acetoxymethyl-, Propionyloxymethyl-, 2-Acetoxyäthyl- oder Pivaloyloxymethylgruppe oder die Phthalidyl- oder Indanylgruppe.

Bedeutet E ein Wasserstoffatom, so fallen unter den Anspruch auch pharmazeutisch verträgliche Salze mit anorganischen oder organischen Basen, wie z. B. die Alkali- oder Erdalkalisalze, beispielsweise die Natrium-, Kalium-, Magnesium- oder Calciumsalze, die Ammoniumsalze oder organische Aminsalze, z. B. mit Triäthylamin oder Dicyclohexylamin.

Bevorzugte Verbindungen der allgemeinen Formel I sind diejenigen, in der

A die Phenyl-, p-Hydroxyphenyl-, 2-Furyl- oder 2-Thienylgruppe und

R ein Wasserstoffatom, die Cyclopropylgruppe, eine gegebenenfalls durch eine Alkanoylgruppe mit 1 bis 4 Kohlenstoffatomen, durch eine Trifluoracetyl-, Cyclohexanoyl-, Benzoyl-, Methylsulfonyl- oder p-Toluolsulfonylgruppe substituierte Aminogruppe, eine Monoalkylaminogruppe mit 1 bis 4 Kohlenstoffatomen, die gegebenenfalls durch eine Hydroxygruppe substituiert sein kann, die 4-Hydroxycyclohexylaminogruppe, eine gegebenenfalls in 3-Stellung durch eine Methyl-, Äthyl-, Cyclohexyl- oder Phenylgruppe substituierte Ureidogruppe, die Hydroxy-, Mercapto-, Methylmercapto-, Dimethylamino-, Äthoxycarbonylamino-, N-Acetylmethylamino-, oder Morpholinogruppe bedeuten und X, D, Het und E die oben angegebenen Bedeutungen haben.

Besonders bevorzugte Verbindungen der allgemeinen Formel I sind jedoch diejenigen, in der
A die Phenyl- oder p-Hydroxyphenylgruppe und
R ein Wasserstoffatom, die Hydroxy-, Formylamino-, Acetylamino-, Propionylamino-, Amino-, Methylamino-, Dimethylamino- oder Ureidogruppe bedeuten und X die oben angegebene Bedeutung hat, worin
D ein Wasserstoffatom, die Acetoxy-, 3-Methyl-1,2,4-thiadiazol-5-yl- oder 1-Methyl-tetrazol-5-yl-gruppe und
E ein Wasserstoffatom oder die Pivaloyloxymethylgruppe bedeuten.

Die Verbindungen der allgemeinen Formel I können bezüglich des Chiralitätszentrums $C^+$ in den beiden möglichen R- und S-Konfigurationen, jedoch auch als ein Gemisch dieser beiden Konfigurationen vorliegen. Bevorzugt sind solche Verbindungen, für welche die D = R-Konfiguration zutrifft.

Die Verbindungen der allgemeinen Formel I zeigen ein breites Wirkungsspektrum gegen grampositive und gramnegative Bakterien bei einer sehr guten Verträglichkeit beim Menschen.

Die Verbindungen der allgemeinen Formel I lassen sich wie folgt darstellen:

1. Umsetzung einer Verbindung der allgemeinen Formel

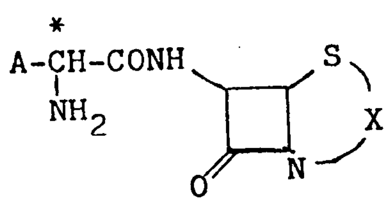

, (II)

in der A und X die oben angegebenen Bedeutungen haben, mit einem Pyrimidinderivat der allgemeinen Formel

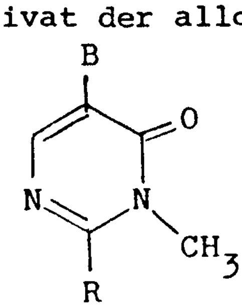

,(III)

in der

R wie eingangs definiert ist und

B die -N=CO oder -NHCO-Hal-Gruppe darstellt, wobei Hal ein Halogenatom, insbesondere ein Chlor- oder Bromatom, bedeutet, oder dessen Gemischen.

Bedeutet E im Rest X der allgemeinen Formel II ein Wasserstoffatom, so können die Ausgangsprodukte der allgemeinen Formel II in Form ihrer anorganischen oder organischen Salze, z. B. als Triäthylammoniumsalz oder Natriumsalz, eingesetzt werden. Die Reaktion kann dann in beliebigen Mischungen von Wasser mit solchen organischen Lösungsmitteln, die mit Wasser mischbar sind, wie Ketonen, z. B. Aceton, cyclische Äther, z. B. Tetrahydrofuran oder Dioxan, Nitrilen, z. B. Acetonitril, Formamiden, z. B. Dimethylformamid, Dimethylsulfoxid oder Alkoholen z. B. Isopropanol oder in Hexametapol durchgeführt werden. Dabei hält man den pH der Reaktionsmischung durch Zusatz von Basen oder Verwendung von Pufferlösungen in einem pH-Bereich von etwa 2,0 bis 9,0, vorzugsweise zwischen pH 6,5 und 8,0. Es ist aber auch möglich, die Reaktion in wasserfreien organischen Lösungsmitteln, z. B. halogenierten Kohlenwasserstoffen, wie Chloroform oder Methylenchlorid unter Zusatz von Basen, vorzugsweise Triäthylamin, Diäthylamin oder N-Äthylpiperidin durchzuführen. Weiterhin läßt sich die Reaktion in einem Gemenge aus Wasser und einem mit Wasser nicht mischbaren Lösungsmittel, wie Äther, z. B. Diäthyläther, halogenierten Kohlenwasserstoffen, z. B. Chloroform oder Methylenchlorid, Schwefelkohlenstoff, Ketonen, z. B. Isobutylmethylketon, Estern, z. B. Essigsäureäthylester, aromatischen Lösungsmitteln, z. B. Benzol ausführen, wobei es zweckmäßig ist, kräftig zu rühren, und den pH-Wert durch Basenzusatz oder Verwendung von Pufferlösungen in einem Bereich von etwa pH 2,0 bis 9,0, vorzugsweise zwischen 6,5 und 8,0, zu halten. Man kann die Reaktion aber auch in Wasser allein in Gegenwart einer organischen oder anorganischen Base oder unter Zusatz von Pufferstoffen durchführen.

Bedeutet dagegen E die Trimethylsilylgruppe, d. h. verwendet man als Ausgangsprodukte für das erfindungsgemäße Verfahren die Silylderivate der Verbindungen der allgemeinen Formel II (z. B. Mono- oder zweckmäßigerweise die sowohl an der Amino- und Carboxylgruppe silylierten Di-trimethylsilylderivate) und setzt sie mit Verbindungen der allgemeinen Formel III um, so arbeitet man im allgemeinen zweckmäßigerweise in wasser- und hydroxyl-gruppenfreien Lösungsmitteln, beispielsweise in halogenierten Kohlenwasserstoffen, z. B. Methylenchlorid oder Chloroform, Benzol, Tetrahydrofuran, Aceton oder Dimethylformamid usw. Der Zusatz von Basen ist hierbei nicht notwendig, kann jedoch in einzelnen Fällen vorteilhaft sein, um die Ausbeute und Reinheit der Produkte zu verbessern. Als gegebenenfalls zugesetzte Basen werden zweckmäßigerweise tertiäre aliphatische oder aromatische Amine, wie Pyridin oder Triäthylamin, oder durch sterische Hinderung schweracylierbare sekundäre Amine, wie Dicyclohexylamin, benützt.

Steht E für eine andere der oben genannten in vitro oder in vivo leicht spaltbaren Schutzgruppppen, z. B. die Benzhydrylgruppe oder die Pivaloyloxymethylgruppe, so empfiehlt es sich ebenfalls in einem aprotischen Lösungsmittel zu arbeiten, z. B. in absolutem Methylenchlorid, Chloroform, Tetrahydrofuran oder Dimethylformamid.

Die Menge der verwendeten Basen ist z. B. durch die gewünschte Einhaltung eines bestimmten pH-Wertes festgelegt. Wo eine pH-Messung und Einstellung nicht erfolgt oder wegen des Fehlens von ausreichenden Mengen Wasser im Verdünnungsmittel nicht möglich oder nicht sinnvoll ist, werden im Falle der Verwendung der nichtsilylierten Verbindungen der allgemeinen Formel II vorzugsweise 1,0 bis 2,0 Moläquivalente Basen eingesetzt. Im Falle der Verwendung der silylierten Verbindungen verwendet man vorzugsweise bis zu einem Moläquivalent Base.

Als Basen können prinzipiell alle in der organischen Chemie üblicherweise verwendeten organischen und anorganischen Basen verwendet werden, wie Alkali- und Erdalkalihydroxide, Erdalkalioxide, Alkali- und Erdalkalicarbonate und Hydrogencarbonate, Ammoniak, primäre, sekundäre und tertiäre aliphatische und aromatische Amine sowie heterocyclische Basen. Beispielhaft seien Natrium-, Kalium- und Calziumhydroxid, Calziumoxid, Natrium- und Kaliumcarbonat, Natrium- und Kaliumhydrogencarbonat, Äthylamin, Methyl-äthylamin, Triäthylamin, Hydroxyäthylamin, Anilin, Dimethylanilin, Pyridin und Piperidin genannt. Bei Verwendung der silylierten Ausgangsstoffe sollten jedoch die oben angegebenen Einschränkungen bezüglich der Art der Basen beachtet werden.

Als Puffersysteme könne alle üblichen Puffermischungen verwendet werden, z. B. Phosphatpuffer, Citratpuffer und Tris-(hydroxymethyl)amino-methan-Puffer.

Die Reaktionstemperaturen können in einem größeren Bereich variiert werden. Im allgemeinen arbeitet man zwischen etwa -20 und etwa +50°C, vorzugsweise zwischen 0 und +20°C.

Die Reaktionspartner der allgemeinen Formeln II und III können von vornherein in äquimolaren Mengen miteinander zur Reaktion gebracht werden. Es kann aber in einzelnen Fällen durchaus zweckmäßig sein, einen der beiden Reaktionspartner im Überschuß zu verwenden, um sich damit die Reinigung des Endproduktes zu erleichtern oder um die Ausbeute zu steigern.

2. Durch Umsetzung von Ureidocarbonsäuren der allgemeinen Formel

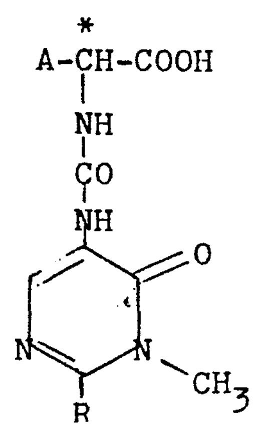

,(IV)

in der A und R die oben angegebenen Bedeutungen besitzen, ihren Salzen oder reaktiven Derivaten, mit Verbindungen der allgemeinen Formel

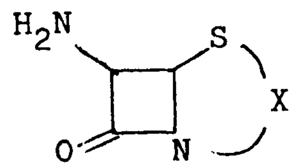

,(V)

in der X die oben angegebenen Bedeutungen hat.

Als reaktive Derivate der Ureidocarbonsäuren der allgemeinen Formel IV kommen beispielsweise deren Säureanhydride wie z. B. die, die sich von Chlorameisensäureestern, z. B. Chlorameisensäureäthyl- oder -isobutylester, ableiten, oder deren reaktive Ester, wie der p-Nitrophenylester oder der N-Hydroxysuccinimidester, oder deren reaktive Amide, wie das N-Carbonylimidazol, aber auch deren Säurehalogenide, wie das entsprechende Säurechlorid oder deren Säureazide in Frage.

Prinzipiell können jedoch alle Verknüpfungsmethoden, wie sie aus der ß-Lactamchemie bekannt sind, verwendet werden.

Die 7-Aminocephalosporansäure oder Penicillansäurederivate der allgemeinen Formel V werden vorteilhafterweise in Form eines in vitro oder in vivo leicht spaltbaren Derivates eingesetzt. Hierfür kommen z. B. die Verbindungen der allgemeinen Formel V in Frage, bei denen E die eingangs gegebenen Bedeutungen mit Ausnahme der eines Wasserstoffatoms besitzt; besonders bevorzugte Derivate sind der Benzhydrylester, der t-Butylester, der Trimethylsilylester oder das N,O-Bis-trimethylsilylderivat.

Die Ureidocarbonsäure, ihre Salze oder ihre reaktiven Derivate werden mit den 7-Aminocephalosporan- oder 6-Aminopenicillansäurederivaten in einem Lösungsmittel bei Temperaturen zwischen $-40^{o}C$ und $+40^{o}C$, gegebenenfalls in Gegenwart einer Base, umgesetzt. Wird z. B. ein Anhydrid der Ureidocarbonsäure, beispielsweise das Anhydrid mit dem Äthylchloroformiat, eingesetzt, so wird die Reaktion unter Kühlung, beispielsweise bei $-10^{o}C$

bis $+10^{o}C$ in Gegenwart eines tertiären Amins, wie Triäthylamin oder N,N-Dimethylanilin, in einem Lösungsmittel, wie Aceton, Tetrahydrofuran, Dimethylformamid, Chloroform, Dichlormethan, Hexametapol oder in einem Gemisch dieser Lösungsmittel, durchgeführt. Setzt man beispielsweise einen N-Hydroxysuccinimidester der Ureidocarbonsäure mit Derivaten der allgemeinen Formel V um, so wird die Reaktion vorzugsweise bei O bis $20^{o}C$ in Anwesenheit einer Base, wie z. B. Triäthylamin, in einem Lösungsmittel wie Dimethylformamid, Dichlormethan, Dioxan oder in einem Gemisch solcher Lösungsmittel durchgeführt.

Die Umsetzung einer Ureidocarbonsäure der allgemeinen Formel IV selbst oder ihrer Salze mit Verbindungen der allgemeinen Formel V erfolgt vorteilhafterweise in Gegenwart eines Kondensationsmittels, z. B. in Gegenwart von N,N'Dicyclohexylcarbodiimid.

Die nach den Verfahren 1 oder 2 hergestellten erfindungsgemäßen Verbindungen, in denen E für eine in vitro leicht abspaltbare Schutzgruppe steht, können nach bekannten Methoden der Cephalosporin- oder Penicillinchemie in die freien Carbonsäuren (E = Wasserstoff) der allgemeinen Formel I überführt werden. So kann beispielsweise die Trimethylsilylgruppe leicht durch wäßrige Hydrolyse entfernt werden oder die Benzhydrylgruppe z. B. durch hydrolytische Spaltung mit Trifluoressigsäure. Diese Eliminierung der Schutzgruppen sind allgemein bekannt.

Ebenso können die Antibiotika der Formel I, worin E ein Wasserstoffatom bedeutet, in Acyloxyalkylester, worin E z. B. einen Pivaloyloxymethylrest

$$-CH_2-O\underset{\underset{O}{\|}}{C}-C(CH_3)_3$$

bedeutet, überführt werden, indem man ein Alkalisalz der freien Carbonsäure, beispielsweise ein Natrium- oder Kaliumsalz, mit einem Pivaloyloxymethylhalogenid der Formel

$$Hal-CH_2-O-\underset{\underset{O}{\|}}{C}-C(CH_3)_3$$

worin Hal für Chlor, Brom oder Jod steht, umsetzt. Weitere geeignete Acyloxyalkylhalogenide sind z. B. Chlormethylacetat, Brommethylpropionat oder 1-Bromäthylacetat.

Die Herstellung der Acyloxyalkylester der Formel I wird vorgenommen, indem man das jeweilige Alkalisalz der Stammsäure in einem inerten Lösungsmittel mit einem leichten molaren Überschuß des Jod- Brom- oder Chloralkylesters, wie Pivaloyloxymethyljodid, bei Raumtemperatur oder leicht erhöhter Temperatur bis zu etwa 40 bis 45°C, umsetzt. Als Lösungsmittel können beispielsweise Aceton, Tetrahydrofuran, Dioxan, Dimethylformamid oder Methylenchlorid verwendet werden.

Die Aufarbeitung der nach den genannten Verfahren erhaltenen Reaktionsgemische nach erfolgter Umsetzung wird nach den bei ß-Lactam-Anitibiotika gebräuchlichen Methoden vorgenommen; dasselbe gilt für die Isolierung und Reinigung der Endprodukte, beispielsweise für die Freisetzung der Säure aus ihren Salzen und die Überführung der freien Säure in andere Salze mittels anorganischer oder organischer Basen. Für die Herstellung der Kalium- oder Natriumsalze bewährt sich besonders die Fällung dieser Salze aus einer alkoholisch-ätherischen Lösung der freien Säure durch die Zugabe von Kalium- oder Natrium-2-äthylhexanoat.

Die Ausgangsstoffe der Formel II (z. B. Ampicillin, Amoxycillin, Epicillin, Cefaloglycin, Cefalexin sowie ihre in vivo leicht spaltbaren Ester) sind bekannt oder können in Analogie zu bekannten Stoffen nach an sich bekannten Methoden hergestellt werden, z. B. durch Acylierung der weitgehend bekannten Amino-lactame der Formel IV und, falls erwünscht, anschließende Umsetzung so erhaltener Cephalosporansäurederivate der Formel II ($Y = -OCOCH_3$) mit Thiolen der Formel Het-SH.

Die Ausgangsstoffe der allgemeinen Formel III können beispielsweise durch Umsetzung der entsprechenden 5-Aminopyrimidine der allgemeinen Formel

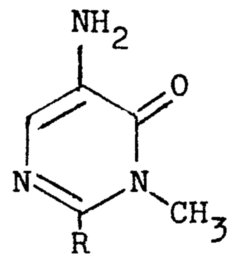

,(VI)

in der R wie oben definiert ist, mit Phosgen gewonnen werden. Diese Umsetzung erfolgt vorzugsweise in einem inerten Lösungsmittel, wie Tetrahydrofuran, Methylenchlorid oder Chloroform, bei Temperaturen zwischen -40°C und +60°C, vorzugsweise zwischen -10°C und +20°C. Dabei empfiehlt es sich, den entstehenden Chlorwasserstoff durch äquimolare Mengen einer inerten organischen Base, wie Triäthylamin oder Pyridin, zu binden. Als Lösungsmittel kann auch Pyridin im Überschuß verwendet werden. Sollten die betreffenden Aminopyrimidine der allgemeinen Formel VI in einem der erwähnten Lösungsmittel schwer löslich sein, kann die Phosgenierung auch in heterogener Phase durchgeführt werden. Desweiteren können die Aminopyrimidine der allgemeinen Formel VI durch Behandlung mit einem Silylierungsmittel, wie Hexamethyldisilazan oder Trimethylchlorsilan/Triäthylamin, in ein im allgemeinen in den erwähnten Lösungsmitteln sehr leicht lösliches, einfach oder, entsprechend den vorhandenen austauschbaren Wasserstoffatomen, mehrfach silyliertes Aminopyrimidin überführt werden, das mit Phosgen dann zu den entsprechenden Verbindungen der allgemeinen Formel III reagiert. Je nach der Art des Lösungsmittels, der Höhe der Temperatur, der Menge und Art der eingesetzten Base entsteht entweder überwiegend das entsprechende Isocyanat oder Carbaminsäurehalogenid oder ein Gemisch dieser beiden Verbindungen.

Die durch Phosgenierung entstandenen Ausgangsprodukte der allgemeinen Formel III bzw. deren Gemische sind in den obenerwähnten Lösungsmitteln leicht löslich und können, nach Entfernung des überschüssigen Phosgens, ohne weitere Reinigung mit den entsprechenden Penicillinderivaten der allgemeinen Formel II direkt umgesetzt werden.

Die 5-Aminopyrimidine der allgemeinen Formel VI sind zum Teil literaturbekannt oder lassen sich analog bekannter Methoden darstellen (vgl. A. Weissberger, The Chemistry of Heterocyclic Compounds, The Pyrimidines, John Wiley, New York). So wird z.B. 3-Methyl-5-nitro-uracil von D.J. Brown et. al. (siehe J.Chem. Soc 1955, 211) beschrieben. Diese Verbindung läßt sich entweder durch katalytische Hydrierung oder besser mit Raney-Nickel/Hydrazin (T.C. Thurber, C.B. Townsend, J. Het. Chem. 1975, S. 711) zur 5-Aminoverbindung reduzieren. Brown et al. (siehe J. Chem. Soc. 1955, 211) stellen aus 2-Thiouracil auch 1.6-Dihydro-1-methyl-2-methylthio-6-oxypyrimidin her. Diese Verbindung läßt sich für die Synthese einer ganzen Anzahl der 5-Aminopyrimidine der allgemeinen Formel VIV als Ausgangsprodukt verwenden, wie folgendes Formelschema an Hand einiger Beispiele zeigt:

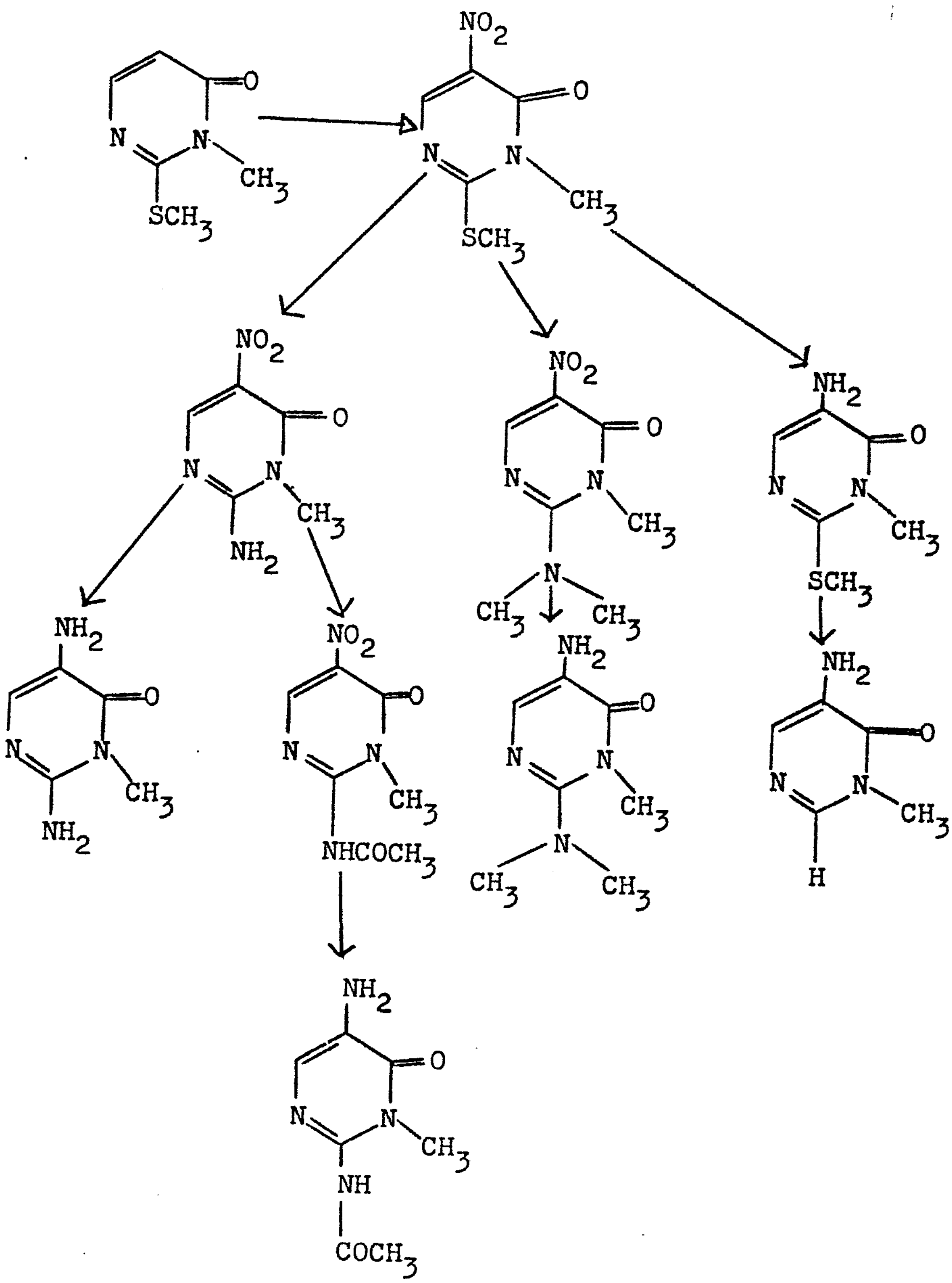

Soweit die Ausgangsprodukte der allgemeinen Formel VI nicht literaturbekannt sind, wird ihre Darstellung in den Beispielen angegeben.

Als typische Vertreter der eingesetzten Pyrimidine der allgemeinen Formel VI seien genannt:

5-Amino-3-methyl-4-oxo-3.4-dihydropyrimidin
5-Amino-2.3-dimethyl-4-oxo-3.4-dihydropyrimidin
2.5-Diamino-3-methyl-4-oxo-3.4-dihydropyrimidin
5-Amino-3-methyl-4-oxo-2-methylmercapto-3.4-dihydropyrimidin
5-Amino-3-methyl-4-oxo-2-mercapto-3.4-dihydropyrimidin
5-Amino-3-methyl-4-oxo-2-methylamino-3.4-dihydro pyrimidin
5-Amino-3-methyl-4-oxo-2-dimethylamino-3.4-dihydropyrimidin
5-Amino-3-methyl-4-oxo-2-anilino-3.4-dihydropyrimidin
5-Amino-3-methyl-4-oxo-2-morpholino-3.4-dihydropyrimidin
5-Amino-3-methyl-4-oxo-2-methylpiperazino-3.4-dihydropyrimidin
5-Amino-3-methyl-4-oxo-2-formylpiperazino-3.4-dihydropyrimidin
5-Amino-3-methyl-4-oxo-2-formylamino-3.4-dihydropyrimidin
5-Amino-3-methyl-4-oxo-2-acetylamino-3.4-dihydropyrimidin
5-Amino-3-methyl-4-oxo-2-propionylamino-3.4-dihydropyrimidin
5-Amino-3-methyl-4-oxo-2-methoxy-3.4-dihydropyrimidin
5-Amino-3-methyl-4-oxo-2-äthoxy-3.4-dihydropyrimidin
5-Amino-3-methyl-4-oxo-2-trifluoracetylamino-3.4-dihydropyrimidin
5-Amino-3-methyl-4-oxo-2-phenylureido-3.4-dihydropyrimidin
5-Amino-3-methyl-4-oxo-2-methylureido-3.4-dihydropyrimidin
5-Amino-3-methyl-4-oxo-2-ureido-3.4-dihydropyrimidin
5-Amino-3-methyl-4-oxo-2-methylsulfonyl-3.4-dihydropyrimidin
5-Amino-2-(N-methyl-N-acetyl)amino-4-oxo-3.4-dihydropyrimidin
5-Amino-3-methyl-2.4-dioxo-1.2-3.4-tetrahydropyrimidin
5-Amino-3-methyl-4-oxo-2-(3'-hydroxypropylamino)-3,4-dihydropyrimidin
5-Amino-3-methyl-4-oxo-2-(4'-hydroxy-cyclohexylamino)-3.4-dihydro-pyrimidin.

Die Ureidocarbonsäuren der allgemeinen Formel IV lassen sich durch Umsetzung der Pyrimidinderivate der allgemeinen Formel III mit Glycinderivaten der allgemeinen Formel

$$A-\overset{+}{\underset{\underset{NH_2}{|}}{C}}-COOH \qquad , VII$$

in der A wie oben definiert ist, erhalten. Die Umsetzung erfolgt bei Temperaturen zwischen $-20^{o}C$ und $+40^{o}C$, vorzugsweise zwischen $0^{o}C$ und $+20^{o}C$, in einem Lösungsmittel, Als Lösungsmittel können hierbei z. B. Gemische von Wasser und organischen Lösungsmitteln, die mit Wasser mischbar sind, verwendet werden, beispielsweise Aceton, Tetrahydrofuran, Dioxan, Acetonitril, Dimethylformamid, Äthanol, Dimethylsulfoxid. Gegebenenfalls wird die Verwendung eines halogenwasserstoffbindenden Mittels notwendig, als solche eignen sich beispielsweise Trialkylamine, wie Triäthylamin, oder anorganische Basen, wie verdünnte Natronlauge.

Es wurde gefunden, daß die Verbindungen der allgemeinen Formel I wertvolle pharmakologische Eigenschaften bei guter Verträglichkeit besitzen. Die erfindungsgemäßen Wirkstoffe können daher zur Prophylaxe und Chemotherapie von lokalen und systemischen Infektionen in der Human- und Tiermedizin verwendet werden. Als Krankheiten, die durch die erfindungsgemäßen Verbindungen verhindert bzw. geheilt werden können, seien beispielsweise solche der Atmungswege, des Rachenraumes und der Harnwege genannt; die Verbindungen wirken insbesondere gegen Pharyngitis, Pneumonie, Peritonitis, Pyelonephritis, Otitis, Cystitis, Endocarditis, Bronchitis, Arthritis und allgemeine systemische Infektionen. Weiter können diese Verbindungen als Stoffe zur Konservierung von anorganischen und organischen Materialien verwendet werden, besonders von organischen Materialien, wie Polymeren, Schmiermittel, Farben, Fasern, Leder, Papier und Holz sowie von Lebensmitteln.

Mit diesen Antibiotika können beispielsweise lokale und/oder systemische Erkrankungen behandelt und/oder verhindert werden, die durch die folgenden Erreger oder durch Mischungen der folgenden Erreger verursacht werden:
Micrococcaceae, wie Staphylokokken;
Lactobacteriaceae, wie Streptokokken;
Neisseriaceae, wie Neisserien;
Corynebacteriaceae, wie Corynebakterien;
Enterobacteriaceae, wie Escherichiae-Bakterien der Coli-Gruppe,
Klebsiella-Bakterien, z.B. K. pneumonia;
Proteae-Bakterien der Proteus-Gruppe z.B. Proteus vulgaris;
Salmonella-Bakterien, z.B. S.thyphimurium;
Shigella-Bakterien, z.B. Shigella dysenteriae,
Pseudomonas-Bakterien, z.B. Pseudomonas aeruginosa;
Aeromonas-Bakterien, z.B. Aeromonas lique faciens;
Spirillaceae, wie Vibrio-Bakterien, z.B. Vibrio cholerae;
Parvobacteriaceae oder Brucellaceae, wie Pasteurella-Bakterien;
Brucella-Bakterien, z.B. Brucella abortus;
Haemophilus-Bakterien, z.B. Haemophilus influenzae;
Bordetella-Bakerien, z.B. Bordetella pertussis;
Moraxella-Bakterien, z.B. Moraxella lacunata;
Bacteroidaceae, wie Bacteroides-Bakterien;
Fusiforme-Bakterien, z.B. Fusobacterium fusiforme;
Sphaerophorus-Bakterien, z.B. Sphaerophorus necrophorus;
Bacillaceae, wie aerobe Sporenbildner, z.B. Bacillus anthracis;
anaerobe Sporenbildner-Chlostridien, z.B. Chlostridium perfringens;
Spirochaetaceae, wie Borrelia-Bakterien;
Treponema-Bakterien, z.B. Treponema pallidum;
Leptospira-Bakterien, wie Leptospira interrogans.

Die obige Aufzählung von Erregern ist lediglich beispielhaft und keineswegs beschränkend aufzufassen.

Dies wird dadurch ermöglicht, daß die Verbindungen der allgemeinen Formel I sowohl in vitro als auch in vivo gegen schädliche Mikroorganismen, insbesondere gegen grampositive und gramnegative Bakterien sehr stark wirken, wobei sie sich besonders durch ein breites Wirkungsspektrum auszeichnen. Daneben zeigen die Verbindungen der allgemeinen Formel I nach oraler und parenteraler Gabe hohe Spiegel in Gewebe, Serum Organen und im Urin.

Die filgenden Verbindungen der allgemeinen Formel I, in der A und R die in der Tabelle genannten Bedeutungen aufweisen, zeigen eine besonders gute antibakterielle Wirksamkeit:

<u>Tabelle:</u>

X =

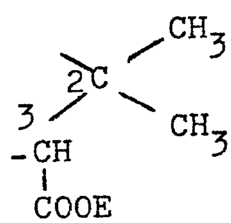

Penicillinderivate

| A | R | E |
|---|---|---|
| Phenyl | H | H |
| p-Hydroxyphenyl | H | H |
| Cyclohexa-1,4-dien-1-yl | H | H |
| Phenyl | OH | H |
| p-Hydroxyphenyl | OH | H |
| Cyclohexa-1,4-dien-1-yl | OH | H |
| p-Hydroxyphenyl | OH | $-CH_2OCOC(CH_3)_3$ |
| Phenyl | NHCHO | H |
| p-Hydroxyphenyl | NHCHO | H |
| p-Hydroxyphenyl | NHCHO | $-CH_2OCOC(CH_3)_3$ |
| Phenyl | $NHCOCH_3$ | H |
| p-Hydroxyphenyl | $NHCOCH_3$ | H |
| p-Hydroxyphenyl | $NHCOCH_3$ | $-CH_2OCOC(CH_3)_3$ |
| Phenyl | $NHCOC_2H_5$ | H |
| p-Hydroxyphenyl | $NHCOC_2H_5$ | H |
| Phenyl | $NH_2$ | H |
| p-Hydroxyphenyl | $NH_2$ | H |

| | | |
|---|---|---|
| p-Hydroxyphenyl | $NH_2$ | $-CH_2OCOC(CH_3)_3$ |
| Phenyl | $N(CH_3)_2$ | H |
| p-Hydroxyphenyl | $N(CH_3)_2$ | H |
| Phenyl | $NHCONH_2$ | H |
| p-Hydroxyphenyl | $NHCONH_2$ | H |
| p-Hydroxyphenyl | $NHSO_2CH_3$ | H |
| p-Hydroxyphenyl | SH | H |

Tabelle 2

Cephalorporinderivate X = 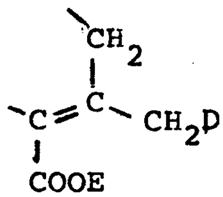

| A | R | D | E |
|---|---|---|---|
| Phenyl | OH | $OCOCH_3$ | H |
| p-Hydroxyphenyl | OH | $OCOCH_3$ | H |
| Phenyl | OH | $CH_3$ | H |
| p-Hydroxyphenyl | OH | $-OCOCH_3$ | $-CH_2OCOC(CH_3)_3$ |
| p-Hydroxyphenyl | OH | 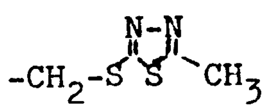 | H |
| p-Hydroxyphenyl | OH | 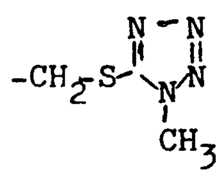 | H |
| p-Hydroxyphenyl | OH | 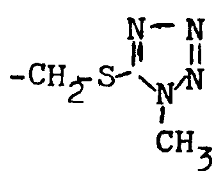 | $-CH_2OCOC(CH_3)_3$ |
| 2-Furyl | OH | 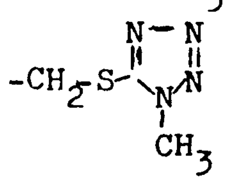 | H |

| | | | |
|---|---|---|---|
| 2-Thienyl | OH | 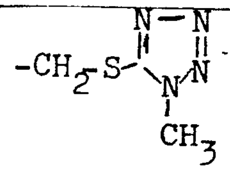 | H |
| p-Hydroxyphenyl | NHCHO | 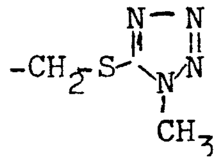 | H |
| p-Hydroxyphenyl | $NHCOCH_3$ | 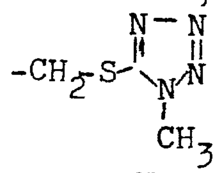 | H |
| p-Hydroxyphenyl | $NHCOCH_3$ | 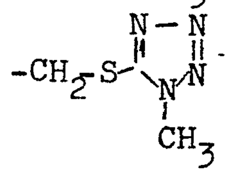 | $-CH_2OCOC(CH_3)_3$ |
| Phenyl | $NH_2$ | $OCOCH_3$ | H |
| p-Hydroxyphenyl | $NHCH_3$ | 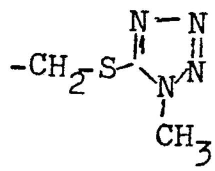 | H |
| p-Hydroxyphenyl | $NHCONH_2$ | 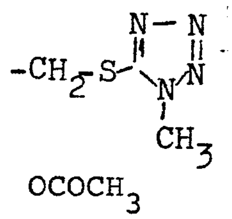 | H |
| Phenyl | $NHCONH_2$ | $OCOCH_3$ | H |
| p-Hydroxyphenyl | $NHSO_2CH_3$ | 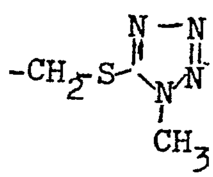 | |

Die Untersuchungen auf die antibakterielle Wirksamkeit in vitro wurde nach dem Agar-Diffusionstest und nach dem Reihenverdünnungstest in Anlehnung an die von P. Klein in

"Bakteriologische Grundlagen der Chemotherapeutischen Laboratoriumspraxis", Springerverlag 1957, Seiten 53 bis 76 und 87 bis 109, beschriebenen Methodik durchgeführt.

Für D-α-[3-(3,4-Dihydro-3-methyl-2-hydroxy-4-oxo-5-pyrimidyl)-ureido]-p-hydroxybenzyl-penicillin-Natrium (Verbindung 1) und für Natrium-7-{D-α-[3-(3,4-Dihydro-2-hydroxy-3-methyl-4-oxo-5-pyrimidyl)-ureido]-p-hydroxyphenylacetamido}-3-[(1-methyl-tetrazol-5-yl)-thiomethyl]-ceph-3-em-4-carboxylat (Verbindung 2) wurden folgende MHK-Werte gegen einige gramnegative Keime gemessen:

| | E.coli ATCC 9637 | E.coli ATCC 11775 | E.coli 12593/74 | Pseud.aerug Hbg. | Pseud.aerug. ATCC 10145 | Klebs. pneum. ATCC 10031 | Klebs. pneum. 272 |
|---|---|---|---|---|---|---|---|
| 1 | 2,5 | 2,5 | >80 | 5 | 5 | 40-80 | 40-80 |
| 2 | 1,25 | 1,25 | 5 | 20 | 20-40 | 1,25 | 2,5 |

| | Proteus mirab. | Proteus Vulgaris | Ser. marcescens ATCC 13880 |
|---|---|---|---|
| 1 | 1,25 | 10 | 2,5 |
| 2 | 1,25 | 10 | 10 |

Weiter wurde gefunden, daß mehrere der in den obigen Tabellen erwähnten Verbindungen der allgemeinen Formel I z. Teil noch in Konzentrationen von < 5 µg/ml gegen Escherichia coli und < 10 µg/ml gegen Pseudomonas aeruginosa und Klebsiella pneumoniae wirksam sind. Weiterhin wurden sehr gute Wirkungen z. B. gegen Proteus vulgaris, Proteus mirabilis und Serratia marcescens festgestellt. Die Verbindungen wirken aber auch

gegen eine Reihe anderer Keime, die gegen andere Penicilline und Cephalosporine resistent sind.

Die gute in vitro Aktivität konnte auch in vivo bestätigt werden. Dabei wurde die $ED_{50}$ der oben genannten Verbindungen 1 und 2 bei einer intraperitonealen E. coli-Infektion an Albino-Mäusen vom Stamm NMRI bestimmt. Am ersten Tag wurde dreimal behandelt (erstmals 1 h nach Infektion) und 2 weitere Tage zweimalig. Für die Verbindung 1 ergab sich bei einer Infektion mit E. coli des Stammes ATCC 11 775 eine $ED_{50}$ von 7 mg/kg bei subcutaner Applikation, für die Verbindung 2 eine $ED_{50}$ von 3,3 mg/kg.

Die Verbindungen der allgemeinen Formel I zeichnen sich weiter durch hohe Verträglichkeit aus. Selbst bei Dosierungen von 2 g/kg konnten an Test-Mäusen keine schädlichen Nebenwirkungen beobachtet werden.

Es ist eine weitere Aufgabe der vorliegenden Erfindung, pharmazeutische Mittel zu schaffen, die bei der Behandlung infektiöser Krankheiten sowohl beim Menschen als auch beim Tier wertvoll sind.

Als bevorzugte pharmazeutische Zubereitungen seien Tabletten, Dragees, Kapseln, Granulate, Suppositorien, Lösungen, Suspensionen, Emulsionen, Salben, Gele, Cremes, Puder und Sprays genannt. Vorteilhafterweise wird der Wirkstoff in der Human- oder Tiermedizin oder ein Gemisch der verschiedenen Wirkstoffe der allgemeinen Formel I in einer Dosierung zwischen 5 und 500, vorzugsweise 10-200 mg/kg Körpergewicht je 24 Stunden verabreicht, gegebenenfalls in Form mehrerer Einzelgaben. Eine Einzelgabe enthält den oder die erfindungsgemäßen Wirkstoffe, vorzugsweise in Mengen von etwa 1 bis etwa 250, insbesondere 10 bis 60 mg/kg Körpergewicht. Es kann jedoch erforderlich sein, von den genannten Dosierungen abzuweichen, und zwar in Abhängigkeit von der Art und dem Körpergewicht des zu behandelnden

Objekts, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die erfindungsgemäßen Antibiotika können zum Zweck der Erweiterung des Wirkungsspektrums und um eine Wirkungssteigerung speziell bei ß-lactamasebildenden Bakterien zu erzielen, mit anderen antimikrobiellen Wirkstoffen, z. B. mit penicillinasefesten Penicillinen kombiniert werden. Hierfür eignen sich besonders Oxacillin oder Dicloxacillin. Ferner können die erfindungsgemäßen Penicillinen mit ß-Lactamaseinhibitoren wie z. B. Clavulansäure kombiniert werden.

Die erfindungsgemäßen Antibiotika können zum Zweck der Erweiterung des Wirkungsspektrums und um eine Wirkungssteigerung zu erreichen auch mit Aminoglykosidantibiotica, wie z. B. Gentamicin, Sisomicin, Kanamicin, Amikacin oder Tobramicin kombiniert werden.

Die folgenden Beispiele sollen die Erfindung näher erläutern:

Mit "Ampicillin" ist dasjenige α-Aminobenzylpenicillin, mit "Amoxycillin" dasjenige α-Amino-p-hydroxy-benzylpenicillin und mit "Epicillin" dasjenige α-Amino-α-(1,4-cyclohexadien-1-yl)-methylpenicillin mit der D=R-Konfiguration in der Seitenkette gemeint.

Mit "Cefalexin" ist diejenige 7-(α-Amino-phenylacetamido)-3-methyl-ceph-3-em-4-carbonsäure und mit "Cephaloglycin" diejenige 7-(α-Amino-phenylacetamido)-3-acetoxymethyl-ceph-3-em-4-carbonsäure mit der D=R-Konfiguration in der Seitenkette gemeint.

Beispiel 1

D-α-[3-(3.4-Dihydro-2-hydroxy-3-methyl-4-oxo-5-pyrimidyl)-ureido]-p-hydroxybenzyl-penicillin-Natrium

Zu 6.5 g Phosgen in 50 ml absolutem Tetrahydrofuran wird unter Eiskühlung eine Lösung von 9 g 5-Amino-3-methyluracil (T.C. Thurber and L.B. Townsend, J. Het. Chem. 1975, 711) und 6,4 g Triäthylamin in absolutem Tetrahydrofuran getropft. Man rührt 45 Minuten bei Raumtemperatur nach. Dann wird Triäthylamin-Hydrochlorid unter Stickstoff abfiltriert und die erhaltene Lösung im Vakuum auf etwa 150 ml eingeengt.

25,0 g Amoxycillin-trihydrat werden mit Triäthylamin in 600 ml 80%igem Tetrahydrofuran gelöst. Unter Eiskühlung wird die oben hergestellte Lösung zugetropft, wobei der pH durch Zugabe von Triäthylamin auf 7,5 gehalten wird. Anschließend wird noch 1 Stunde bei Raumtemperatur nachgerührt. Tetrahydrofuran wird im Vakuum abgezogen und die zurückbleibende wäßrige Lösung bei

pH 7,5 zweimal mit Essigesterextrahiert. Anschließend wird mit Essigester überschichtet und unter Eiskühlung mit verdünnter Salzsäure auf pH 2,0 angesäuert. Die saure wäßrige Lösung wird noch zweimal mit Essigester ausgeschüttelt, die vereinigten organischen Extrakte werden mit Magnesiumsulfat getrocknet unß im Vakuum zur Trockne eingeengt.
Rohausbeute der Säure: 25,4 g (80 % der Theorie).
Diese Säure wird in 250 ml absolutem Methanol und 750 ml Äther gelöst und mit Natrium-äthylhexanoat das Natriumsalz ausgefällt.
Ausbeute: 25,5 g (74 % der Theorie),
Rf-Wert: 0,73
IR-Spektrum: 1770, 1715, 1640 $cm^{-1}$;
NMR-Spektrum ($D_2O$): Signale bei ppm 1,4 (3H), 2,5 (3H), 3,2 (3H), 4,2 (1H), 5,3-5,5 (3H), 7,1 (4H), 7,8 (1H)

In analoger Weise wird erhalten:

a) <u>D-α-[3,4-Dihydro-2-hydroxy-3-methyl-4-oxo-5-pyrimidyl)-ureido]-benzylpenicillin-Natrium</u>

hergestellt aus 4 g Ampicillin-trihydrat und dem Umsetzungsprodukt von 1,4 g 5-Amino-3-methyluracil, 1 g Phosgen und 1 g Triäthylamin in Tetrahydrofuran.
Ausbeute: 4,1 g
Rf-Wert: 0,76
IR-Spektrum 1770, 1710, 1650 $cm^{-1}$
NMR-Spektrum ($D_2O$): Signale bei ppm 1,5 (3H), 1,6 (3H), 3,3 (3H), 4,25 (1H), 5,4-5,6 (3H), 7,4 (5H), 7,9 (1H)

b) <u>D-α-[3-(3,4-Dihydro-2-hydroxy-3-methyl-4-oxo-5-pyrimidyl)-ureido]-1,4-cyclohexadienylmethyl-penicillin-Natrium</u>

ausgehend von 1,87 g (0.005 Mol) Epicillin-Natrium und 0,7 g (0.005 Mol) des Amins des Beispiels 1 sowie 500 mg Phosgen und 0,67 ml Triäthylamin.
Ausbeute: 1,93 g (71 %)
IR-Spektrum: 1765, 1710, 1645, 1590 $cm^{-1}$
NMR-Spektrum ($D_2O$): Signale bei ppm 1,6 (3H), 1,7 (3H), 2,5-

3,o (4H), 3,35 (3H), 4,2 (1H), 5,4-5,7 (3H), 4,3 (2H) 4,1 (1H)

Beispiel 2

Natrium-7-{D-α-[3-(3,4-Dihydro-2-hydroxy-3-methyl-4-oxo-5 pyrimidyl)-ureido]-phenylacetamido}-3-acetoxymethyl-ceph-3-em-4-carboxylat

1,41 g 5-Amino-3-methyluracil (0,01 Mol) werden wie in Beispiel 1 mit 1,0 g Phosgen und 1,37 ml Triäthylamin umgesetzt. 4,25 g (0,01 Mol) Cephaloglycin-Dihydrat werden in 80 ml 80%igem wäßrigem Tetrahydrofuran suspendiert und durch zutropfen von Triäthylamin in Lösung gebracht. Zu dieser Lösung wird die oben hergestellte Suspension zugetropft und dabei der pH-Wert durch Triäthylamin bei 7,0-7,5 gehalten. Anschließend wird eine Stunde bei Eisbadkühlung und eine Stunde bei Raumtemperatur nachgerührt. Man fügt 40 ml Wasser hinzu, dampft das Tetrahydrofuran im Vakuum weitgehend ab und schüttelt die zurückbleibene wäßrige Phase bei pH 7,0 zweimal mit Essigsäureäthylester aus. Dann wird mit viel Essigsäureäthylester überschichtet und unter Eiskühlung und Rühren mit 2 n Salzsäure auf pH 2,0 angesäuert. Die organische Schicht wird abgetrennt, mit gesättigter Natriumchloridlösung gewaschen, über Natriumsulfat getrocknet und im Vakuum zur Trockne eingeengt. Das zurückbleibende Festprodukt wird in wenig Methanol mit der entsprechenden Menge Natriumhexanoat gelöst und mit Äther versetzt. Das ausgefallene Produkt wird abgesaugt und getrocknet.

Ausbeute 4,15 g (74 %)

IR-Spektrum: 1760, 1725, 1670, 1610 $cm^{-1}$

NMR-Spektrum (DMSO + $CD_3OD$) Signale bei ppm: 2,05 (s, 3H) 3,15 (s, 3H), 3,45 (m,2H), 4,8 (m, 2 + 1 H), 5,4 (s,1H), 5,75 (d, 1H), 7,45 (5H), 8.05 (1H),

Analog wurden synthetisiert:

a) Natrium-7-{D-α-[3-(3,4-Dihydro-2-hydroxy-3-methyl-4-oxo-5 pyrimidyl)-ureido]-p-hydroxy-phenylacetamido}-3-acetoxymethyl-ceph-3-em-4-carboxylat

ausgehend von 0,7 g (0,005 Mol) des Amins des Beispiels 2 sowie 2,0 g (0,005 Mol) des p-Hydroxyanalogen des Cefaloglycins.
Ausbeute: 2,0 g (72 %)
IR-Spektrum 1765, 1730, 1670, 1610 $cm^{-1}$
NMR-Spektrum (DMSO + $CD_3OD$) Signale bei ppm: 2,10 (s, 3H), 3,15 (s, 3H), 3,50 (m, 2H), 4,4 - 4,9 (m, 2 + 1H), 5,45 (s, 1H), 5,80 (d, 1H), 6,85 (d, 2H), 7,40 (d, 2H), 8,05 (s, 1H),

b) Natrium-7-{D-α-[3-(3,4-Dihydro-2-hydroxy-3-methyl-4-oxo-5 pyrimidyl)-ureido]-phenylacetamido}-3-methyl-ceph-3-em-4-carboxylat

ausgehend von 3,5 g Cefalexinmonohydrat und dem Umsetzungsprodukt von 1,41 g des Pyrimidins des Beispiels 2 mit 1,0 g Phosgen und 1,37 ml Triäthylamin.
Ausbeute: 4,16 g (81 %)
IR-Spektrum 1760, 1660, 1605 $cm^{-1}$
NMR-Spektrum (DMSO + $CD_3OD$) Signale bei ppm: 2,0 (3H), 3,2 (m, 2H + s, 3H), 4,9 (d, 1H), 5,55 (d, 2H), 5,6 (s, 1H), 7,45 (m, 5H), 8,0 (s, 1H).

## Beispiel 3

6-{D-α-[3-(3,4-Dihydro-2-hydroxy-3-methyl-4-oxo-5-pyrimidyl)-ureido]-p-hydroxy-phenylacetamido}-penicillansäure-pivaloyloxymethylester

1,1 g (0,002 Mol) der Verbindung des Beispiels 1 werden in 5 ml Aceton suspendiert. Dazu gibt man eine Lösung von 340 mg

Pivaloyloxymethylchlorid (0,0022 Mol) in 10 ml Aceton, sowie 0,2 ml einer 25 %igen Natriumjodidlösung in Wasser. Man erhitzt 5 Stunden zum Rückfluß, kühlt ab und versetzt mit 15 ml Eiswasser. Es wird abdekantiert und das ausgefallene etwas schmierige Produkt erneut mit 10 ml Eiswasser versetzt und 5 Min. gerührt. Danach wird abgesaugt, das farblose Produkt in Essigester gelöst, mit Wasser, Natriumhydrogencarbonat-Lösung und wieder mit Wasser gewaschen und anschließend im Vakuum getrocknet.
Ausbeute: 800 mg (62 %)
IR-Spektrum: 1770, 1720, 1650 $cm^{-1}$
NMR-Spektrum ($CDCl_3$ + $CD_3OD$) Signale bei ppm: 1,2 (s, 9H), 1,55 (d, 6H), 3,2 (s, 3H), 4,2 (s, 1H), 5,45 (q, 2H), 5,5 (1H), 5,65 (q, 2H), 6,85 (d, 2H), 7,35 (d, 2H), 8,05 (s, 1H).

Analog wurden synthetisiert:

a) <u>7-{D-α-/3-(3,4-Dihydro-2-hydroxy-3-methyl-4-oxo-5-pyrimidyl)-ureido/-p-hydroxy-phenylacetamido}-3-acetoxymethyl-ceph-3-em-4-carbonsäure-pivaloyloxymethylester</u>

ausgehend von dem Cephalosporin des Beispiels 2 a und Pivaloyloxymethylchlorid. Das ausgefallene Produkt wird über eine Silicagelsäule gereinigt. Eluens $CH_2Cl_2/CH_3OH$ 14:1.
IR-Spektrum. 1765, 1710, 1665, 1610 $cm^{-1}$
NMR-Spektrum ($CDCl_3$ + $CD_3OD$) Signale bei ppm: 1,25 (s, 9H), 2,10 (s, 3H), 3,20 (s, 3H), 3,55 (m, 2H), 4,4 - 4,9 (m, 3H), 5,45 (s, 1H), 5,6 - 5,9 (m, 2 + 1H), 6,85 (d, 2H), 7,35 (d, 2H), 8,05 (s, 1H),

<u>Beispiel 4</u>

<u>Natrium-7-{D-α-/3-(3,4-Dihydro-2-hydroxy-3-methyl-4-oxo-5-pyrimidyl)-ureido/-p-hydroxyphenylacetamido}-3-/(2-methyl-1,3,4-thiadiazol-5-yl)-thiomethyl/-ceph-3-em-4-carboxylat</u>

Herstellung analog Beispiel 2 ausgehend von 4,6 g (0,01 Mol) 7-(D-α-Amino-p-hydroxyphenylacetamido)-3-[(2-methyl-1,3,4-thiadiazol-5-yl)-thiomethyl]-ceph-3-em-4-carbonsäure sowie dem Umsetzungsprodukt von 1,41 g (0,01 Mol) des Pyrimidins des Beispiels 1 mit 1,0 g Phosgen und 1,37 ml Triäthylamin.

Bei der Aufarbeitung wird die entstanden Cephem-carbonsäure bei pH 2,0 aus Wasser ausgefällt, abgesaugt und getrocknet.

Ausbeute an Natriumsalz: 4,41 g (68 %)

IR-Spektrum: 1760, 1650, 1610 $cm^{-1}$

NMR-Spektrum: DMSO + $CD_3OD$) Signale bei ppm: 2,7 (s, 3H), 3,25 (s, 3H), 3,4 (m, 2H), 4,3 (überlagert vom Lösemittelpeak), 4,85 (d, 1H), 5,45 (s, 1H), 5,75 (d, 1H), 6,85 (d, 2H), 7,35 (d, 2H), 8,0 (s, 1H).

## Beispiel 5

**Natrium-7-{D-α-[3-(3,4-Dihydro-2-hydroxy-3-methyl-4-oxo-5-pyrimidyl)-ureido]-p-hydroxy-phenylacetamido}-3-[(1-methyl-tetrazol-5-yl)-thiomethyl]-ceph-3-em-4-carboxylat**

a) D-α-[3-(3,4-Dihydro-2-hydroxy-3-methyl-4-oxo-5-pyrimidyl)-ureido]-p-hydroxy-phenylglycin

2,82 g des Pyrimidins des Beispiels 1 werden in der dort angegebenen Weise mit 1,0 g Phosgen und 1,37 ml Triäthylamin umgesetzt. Das Gemisch wird zu einer Lösung von 3,35 g p-Hydroxy-D-phenylglycin in 80 ml 80%igem wäßrigem Tetrahydrofuran und 20 ml 1 n Natronlauge getropft. Der pH wird bei der Zugabe auf 8,0 - 8,5 gehalten. Man rührt 3 Stunden bei Raumtemperatur bei pH 8,0 nach. Dann wird das Tetrahydrofuran im Vakuum abdestilliert und die wäßrige

Lösung zweimal mit Essigester ausgeschüttelt. Mit 2 n Salzsäure wird dann auf pH 2,0 gestellt und das ausgefallene Festprodukt abgesaugt. Nach Trocknen im Vakuum erhält man 4,28 g (64 %) der Ureidocarbonsäure.
NMR-Spektrum. (DMSO + $CD_3OD$) Signale bei ppm: 3,3 (s, 3H), 5,15 (d, 1H), 6,8 (d, 2H), 7,4 (d, 2H), 8,05 (s, 1H)

b) <u>7-{D-α-[3-(3,4-Dihydro-2-hydroxy-3-methyl-4-oxo-5-pyrimidyl)-ureido]-p-hydroxy-phenylacetamido}-3-[(1-methyl-tetrazol-5-yl)thiomethyl]-ceph-3-em-4-carbonsäure-benzhydrylester</u>

3,3 g (0,01 Mol) der unter a) hergestellten Ureidocarbonsäure werden in 120 ml Methylenchlorid gelöst. Unter Eiskühlung fügt man 4,95 g 7-Amino-3-[(2-methyl-tetrazol-5-yl)-thiomethyl]-ceph-3-em-4-carbonsäure-benzhydrylester (0,01 Mol) sowie 2,3 g Dicyclohexylcarbodiimid zu. Man läßt 6 Stunden unter Eiskühlung reagieren und saugt dann den entstandenen Harnstoff ab. Es wird im Vakuum zur Trockne eingeengt und das Rohprodukt über eine Silicagelsäule mit dem Eluens Methylenchlorid/Methanol 10:1 gereinigt.
Ausbeute 5,62 g (71 %)

c) Das unter b) erhalten Produkt wird in 30 ml trockenem Methylenchlorid aufgeschlämmt und mit 20 ml Trifluoressigsäure sowie 5 ml Anisol im Eisbad 45 Min. gerührt. Man gibt zweimal 100 ml Toluol zu und engt im Vakuum jeweils zur Trockne ein. Das entstandene Festprodukt wird in Äther aufgeschlämmt und abgesaugt. In Methanol wurd auf übliche Weise das Natriumsalz hergestellt.
Ausbeute: 4,05 g (86 %)
IR-Spektrum. 1760, 1670, 1610 $cm^{-1}$
NMR-Spektrum (DMSO + $CD_3OD$) Signale bei ppm: 3,2 (s, 3H), 3,45 (m, 2H), 3,95 (s, 3H), 4,3 - 4,4 (vom Lösungsmittelpeak überlagert), 4,9 (d, 1H), 5,4 (s, 1H), 5,5 (d, 1H), 6,7 (d, 2H), 7,3 (d, 2H), 8.05 (s, 1H)

Analog wurden synthetisiert:

Natrium-7-{D-α-[3-(3,4-Dihydro-2-hydroxy-3-methyl-4-oxo-5-pyrimidyl)-ureido]-2-furylacetamido}-3-[(1-methyl-tetrazol-5-yl)thiomethyl]-ceph-3-em-4-carboxylat

Natrium-7-{D-α-[3-(3,4-Dihydro-2-hydroxy-3-methyl-4-oxo-5-pyrimidyl)-ureido]-2-thienylacetamido}-3-[(1-methyl-tetrazol-5-yl)thiomethyl]-ceph-3-em-4-carboxylat

d) 7-{D-α-[3-(3,4-Dihydro-2-hydroxy-3-methyl-4-oxo-5-pyrimidyl)-ureido]-p-hydroxy-phenylacetamido}-3-[(1-methyl-tetrazol-5-yl)-thiomethyl]-ceph-3-em-4-carbonsäure-pivaloyloxymethylester

Dieser Ester wird analog der Vorschrift des Beispiels 3 hergestellt, indem man von 650 mg (0,001 Mol) der Verbindung des Beispiels 5 c und 170 mg Pivaloyloxymethylchlorid ausgeht.
Ausbeute 380 mg (52 %) nach Chromatographie über eine Silicagelsäule ($CH_2Cl_2/CH_3OH$ 10:1).
IR-Spektrum 1770, 1730, 1670, 1610 $cm^{-1}$
NMR-Spektrum ($CDCl_3/CD_3OD$) Signale bei ppm: 1,25 (s, 9H) 3,25 (s, 3H), 3,5 (m, 2H), 3,90 (s, 3H), 4,4 (m, 2H), 4,9 (d, 1H), 5,45 (s, 1H), 5,55 (d, 1H), 5,7 (q, 2H), 6,75 (d, 2H), 7,35 (d, 2H), 8,0 (s, 1H),

## Beispiel 6

a) 3,4-Dihydro-5-nitro-3-methyl-2-methylmercapto-4-oxo-pyrimidin

20 g 3,4-Dihydro-3-methyl-2-methylmercapto-4-oxo-pyrimidin werden 12 Stunden lang bei Raumtemperatur in einem Gemisch aus 80 ml rauchender Salpetersäure und 80 ml konzentrierter Schwefelsäure gerührt. Dann gießt man vorsichtig auf 500 ml Eis, saugt den ausgefallenen Niederschlag ab, wäscht mit Wasser gut aus und trocknet.
Ausbeute: 22,8 g (95 % der Theorie), Schmelzpunkt: 152°C.

0006532

$C_6H_7N_3O_3S$ (201.2)

| | | | | |
|---|---|---|---|---|
| Ber.: | C 35,87 | H 3,51 | N 20,89 | S 15,94 |
| Gef.: | 35,55 | 3,71 | 20,85 | 16,10 |

b) 3.4-Dihydro-5-amino-3-methyl-2-methylmercapto-4-oxo-pyrimidin

3.0 g der gemäß a) erhaltenen Nitroverbindung werden mit 12 g wasserfeuchtem Raney-Nickel 1,5 Stunden auf Rückfluß erhitzt. Man filtriert heiß ab und engt zur Trockne ein. Der ölige Rückstand wird über eine Kieselgelsäule gereinigt (Methylenchlorid/Methanol 10:1). Die Verbindung mit dem höchsten Rf-Wert wird abgetrennt.

Ausbeute: 0,9 g (39 % der Theorie),

$C_6H_9N_3OS$ (171.2)

| | | | | |
|---|---|---|---|---|
| Ber.: | C 42.10 | H 5.28 | N 24.42 | S 18.20 |
| Gef.: | 42.40 | 5.26 | 24.10 | 17.55 |

c) D-α-[3-(3.4-Dihydro-3-methyl-2-methylmercapto-4-oxo-5-pyrimidyl)-ureido]-p-hydroxybenzyl-penicillin-Natrium

Zu 600 mg Phosgen in 10 ml absolutem Tetrahydrofuran wird unter Eiskühlung eine Lösung von 1.0 g 3.4-Dihydro-5-amino-3-methyl-2-methylmercapto-4-oxo-pyrimidin und 600 mg Triäthylamin in Tetrahydrofuran getropft. Das Gemisch wird auf etwa 50 ml eingeengt und unter Eiskühlung zu einer Lösung getropft, die aus 2.5 g Amoxycillin und Triäthylamin in 80 ml 80 %igem Tetrahydrofuran bereitet wurde. Die Aufarbeitung erfolgte analog Beispiel 1.

Ausbeute: 2.35 g (77 % der Theorie),

Rf-Wert: 0,76

IR-Spektrum: 1770, 1650, 1600, 1510 $cm^{-1}$

NMR-Spektrum ($D_2O$): Signale bei ppm 1.4 (3H), 1.5 (3H), 2.5 (3H), 3.3 (3H), 4.2 (1H), 5.2-5.6 (3H), 6.8 (2H), 7.2 (2H), 8.2 (1H)

In analoger Weise wird erhalten:
D-α-[3-(3.4-Dihydro-3-methyl-2-methylmercapto-4-oxo-5-pyrimidyl)-ureido]-1.4-cyclohexadienyl-methylpenicillin-Natrium

## Beispiel 7

### a) 3.4-Dihydro-5-amino-3-methyl-4-oxo-pyrimidin

3.0 g des gemäß Beispiel 6 erhaltenen Ausgangsproduktes werden mit 15 g feuchtem Raney-Nickel so lange in 50 ml Wasser zum Rückfluß erhitzt, bis kein Methylmercaptan mehr entsteht. Es wird vom Raney-Nickel abfiltriert und zur Trockne eingeengt.

Ausbeute: 1.8 g (80 % der Theorie),

$C_5H_6N_3O$ (124.13)

| | | | |
|---|---|---|---|
| Ber.: | C 48.30 | H 4.83 | N 33.85 |
| Gef.: | 48.07 | 4.92 | 32.86 |

### b) D-α-[3-(3.4-Dihydro-3-methyl-4-oxo-5-pyrimidyl)-ureido]-p-hydroxybenzylpenicillin-Natrium

Aus 1.9 g Amoxycillin-trihydrat und 600 mg 3.4-Dihydro-5-amino-3-methyl-4-oxo-pyrimidin. Reaktionsführung und Aufarbeitung erfolgt analog Beispiel 1.

Ausbeute: 1.45 g (59 % der Theorie),

Rf-Wert: 0.61

IR-Spektrum: 1765, 1650, 1610, 1550 $cm^{-1}$

NMR-Spektrum ($CD_3OD$): Signale bei ppm 1.5 (3H), 1.6 (3H), 3.6 (3H), 4.2 (1H), 5.4 (1H), 5.5 (2H), 6.8 (2H), 7.3 (2H), 8.1 (1H), 8.7 (1H)

In analoger Weise wird erhalten:
D-α-[3-(3.4-Dihydro-3-methyl-4-oxo-5-pyrimidyl)-ureido]-benzylpenicillin-Natrium
D-α-[3-(3.4-Dihydro-3-methyl-4-oxo-5-pyrimidyl)-ureido]-1,4-cyclohexadienyl-methylpenicillin-Natrium

0006532

Natrium-7-{D-α-/3-(3.4-Dihydro-3-methyl-4-oxo-5-pyrimidyl)-ureido/-phenylacetamido}-3-acetoxymethyl-ceph-3-em-4-carboxylat

Beispiel 8

a) 3.4-Dihydro-2-amino-5-nitro-3-methyl-4-oxo-pyrimidin

12 g des gemäß Beispiel 6a erhaltenen Nitropyrimidins werden bei Raumtemperatur mit 500 ml methanolischer Ammoniaklösung gerührt. Das Ausgangsprodukt geht in Lösung und es fällt das gewünschte Produkt aus, das abgesaugt und mit kaltem Methanol ausgewaschen wird.

Ausbeute: 8,8 g (96 % der Theorie),

$C_5H_6N_4O_3$ (170.1)

| | | | |
|---|---|---|---|
| Ber.: | C 35.28 | H 3.55 | N 32.94 |
| Gef.: | 35.30 | 3.58 | 32.80 |

b) 3.4-Dihydro-2-acetylamino-5-nitro-3-methyl-4-oxo-pyrimidin

5.1 g des gemäß Beispiel 8a erhaltenen Pyrimidins werden in 50 ml Acetanhydrid vorgelegt und auf Rückfluß erhitzt. Nach etwa 20 Minuten hat sich alles gelöst. Man hält weitere 20 Minuten auf etwa 100°C und läßt dann erkalten. Acetanhydrid wird im Vakuum entfernt. Der ölige Rückstand kristallisiert nach Zugabe von Methanol.

Ausbeute: 5.4 g (85 % der Theorie),

Schmelzpunkt: Zersetzung ab etwa 148°C.

$C_7H_8N_4O_4$ (212.2)

| | | | |
|---|---|---|---|
| Ber.: | C 39.63 | H 3.80 | N 26.41 |
| Gef.: | 39.50 | 3.85 | 26.10 |

In analoger Weise wird erhalten:

3.4-Dihydro-5-nitro-2-trifluoracetylamino-3-methyl-6-oxo-pyrimidin.

Ausbeute: 64 % der Theorie, Schmelzpunkt: 160°C

$C_7H_5F_3N_4O_4$ (266.2)

| | | | |
|---|---|---|---|
| Ber.: | C 31.58 | H 1.90 | N 21.08 |
| Gef.: | 31.30 | 1.95 | 22.05 |

c) 3.4-Dihydro-5-amino-2-acetylamino-3-methyl-4-oxo-pyrimidin

1.1 g der gemäß Beispiel 8b erhaltenen Nitroverbindung werden in 50 ml Dimethylformamid gelöst und mit 0.5 g Palladium/Kohle (5 %) bei Normalbedingungen hydriert. Nach Ende der Wasserstoffaufnahme wird vom Katalysator abfiltriert und das Filtrat eingeengt. Der kristalline Rückstand wird aus Äthanol mit etwas Aktivkohle umkristallisiert.

Ausbeute: 620 mg (68 % der Theorie),
Schmelzpunkt: 192-195°C (Zersetzung)
$C_7H_{10}N_4O_2$ (182.2)

| | | | |
|---|---|---|---|
| Ber.: | C 46.20 | H 5.48 | N 30.75 |
| Gef.: | 46.50 | 5.68 | 30.45 |

In analoger Weise wird erhalten:
3.4-Dihydro-5-amino-2-trifluoracetylamino-3-methyl-4-oxo-pyrimidin
Die Reinigung erfolgt säulenchromatographisch über Kieselgel (Methylenchlorid/Methanol 5:1).
Ausbeute: 37 % der Theorie,
$C_7H_7F_3N_4O_2$

| | | | |
|---|---|---|---|
| Ber.: | C 35.55 | H 2.99 | N 23.75 |
| Gef.: | 35.99 | 3.14 | 23.50 |

Analog werden erhalten:
3.4-Dihydro-5-amino-2-formylamino-3-methyl-4-oxo-pyrimidin
3.4-Dihydro-5-amino-2-isobutyrylamino-3-methyl-4-oxo-pyrimidin
3.4-Dihydro-5-amino-2-butyrylamino-3-methyl-4-oxo-pyrimidin
3.4-Dihydro-5-amino-2-hexanoylamino-3-methyl-4-oxo-pyrimidin
3.4-Dihydro-5-amino-2-cyclohexanoylamino-3-methyl-4-oxo-pyrimidin
3.4-Dihydro-5-amino-2-benzoylamino-3-methyl-4-oxo-pyrimidin
3.4-Dihydro-5-amino-2-methylsulfonylamino-3-methyl-4-oxo-pyrimidin

3.4-Dihydro-5-amino-2-tosylamino-3-methyl-4-oxo-pyrimidin

d) D-α-/3-(3.4-Dihydro-2-acetylamino-3-methyl-4-oxo-5-pyrimidyl)-ureido/-p-hydroxybenzylpenicillin-Natrium

Analog Beispiel 1 aus 3.2 g Amoxycillin-trihydrat, 1.4 g des gemäß Beispiel 8c erhaltenen Amins, das mit 800 mg Phosgen und 800 mg Triäthylamin umgesetzt wurde, in Tetrahydrofuran.
Ausbeute: 2.6 g (66 % der Theorie),
Rf-Wert: 0.62
IR-Spektrum: 1770, 1650, 1600, 1510 $cm^{-1}$
NMR-Spektrum ($D_2O$): Signale bei ppm 1.4 (6H), 2.3 (3H), 3.5 (3H), 4.2 (1H), 5.3 (1H), 5.5 (2H), 6.9 (2H), 7.3 (2H), 8.4 (1H).

In analoger Weise werden erhalten:

D-α-/3-(3.4-Dihydro-2-trifluoracetylamino-3-methyl-4-oxo-5-pyrimidyl)-ureido/-p-hydroxybenzylpenicillin-Natrium
Ausbeute: 52 % der Theorie,
Rf-Wert: 0.54
IR-Spektrum: 1770, 1660, 1540, 1190 $cm^{-1}$
NMR-Spektrum ($D_2O$): Signale bei ppm 1.4 (3H), 1.5 (3H), 3.45 (3H), 4.2 (1H), 5.3 (1H), 5.5 (2H), 6.9 (2H), 7.3 (2H), 8.25 (1H)
sowie:
D-α-/3-(3.4-Dihydro-2-trifluoracetylamino-3-methyl-4-oxo-5-pyrimidyl)-ureido/-benzylpenicillin-Natrium.
D-α-/3-(3.4-Dihydro-2-acetylamino-3-methyl-4-oxo-5-pyrimidyl)-ureido/-benzylpenicillin-Natrium
D-α-/3-(3.4-Dihydro-2-acetylamino-3-methyl-4-oxo-5-pyrimidyl)-ureido/-1.4-cyclohexadienyl-methylpenicillin-Natrium
D-α-/3-(3.4-Dihydro-2-formylamino-3-methyl-4-oxo-5-pyrimidyl)-ureido/-p-hydroxybenzyl-penicillin-Natrium

D-α-[3-(3.4-Dihydro-2-formylamino-3-methyl-4-oxo-5-pyrimidyl)-ureido]-benzylpenicillin-Natrium

D-α-[3-(3.4-Dihydro-2-formylamino-3-methyl-4-oxo-5-pyrimidyl)-ureido]-1.4-cyclohexadienyl-methylpenicillin-Natrium

D-α-[3-(3.4-Dihydro-2-butyrylamino-3-methyl-4-oxo-5-pyrimidyl)-ureido]-p-hydroxybenzylpenicillin-Natrium

D-α-[3-(3.4-Dihydro-2-isobutyryl-3-methyl-4-oxo-5-pyrimidyl)-ureido]-p-hydroxybenzylpenicillin-Natrium

D-α-[3-(3.4-Dihydro-2-isobutyrylamino-3-methyl-4-oxo-5-pyrimidyl)-ureido]-benzylpenicillin-Natrium

D-α-[3-(3.4-Dihydro-2-hexanoyl-3-methyl-4-oxo-5-pyrimidyl)-ureido]-benzylpenicillin-Natrium

D-α-[3-(3.4-Dihydro-2-hexanoyl-3-methyl-4-oxo-5-pyrimidyl)-ureido]-p-hydroxybenzylpenicillin-Natrium

D-α-[3-(3.4-Dihydro-2-benzoylamino-3-methyl-4-oxo-5-pyrimidyl)-ureido]-p-hydroxybenzylpenicillin-Natrium

D-α-[3-(3.4-Dihydro-2-methylsulfonylamino-3-methyl-4-oxo-5-pyrimidyl)-ureido]-p-hydroxybenzylpenicillin-Natrium

D-α-[3-(3.4-Dihydro-2-methylsulfonylamino-3-methyl-4-oxo-5-pyrimidyl)-ureido]-benzylpenicillin-Natrium

D-α-[3-(3.4-Dihydro-2-tosylamino-3-methyl-4-oxo-5-pyrimidyl)-ureido]-p-hydroxybenzylpenicillin-Natrium

## Beispiel 9

Natrium-7-{D-α-[3-(3,4-Dihydro-2-acetylamino-3-methyl-4-oxo-5-pyrimidyl)-ureido]-p-hydroxy-phenylacetamido}-3-[(1-methyl-tetrazol-5-yl)-thiomethyl]-ceph-3-em-4-carboxylat

Herstellung analog Beispiel 5 ausgehend von 1,87 g (0,005 Mol) D-α-[3-(3,4-Dihydro-2-acetylamino-3-methyl-4-oxo-5-pyrimidyl)-ureido]-p-hydroxyphenylglycin sowie 2,5 g (0,005 Mol) des Benzhydrylesters des Beispiels 5 und 1,1 g Dicyclohexylcarbodiimid

Ausbeute nach Abspalten der Schutzgruppe: 2,3 g (64 % der Theorie)

IR-Spektrum: 1765, 1670, 1610 $cm^{-1}$

NMR-Spektrum (DMSO + $CD_3OD$) Signale bei ppm: 2,1 (s, 3H), 3,4 (s, 3H), 3,6 (m, 2H), 3,95 (s, 3H), 4,4 - 4,6 (vom Lösemittel überdeckt), 4,9 (d, 2H), 5,45 (s, 1H), 5,6 (d, 2H), 6,8 (d, 2H), 7,35 (d, 2H), 8,55 (s, 1H)

Analog wurde synthetisiert:
Natrium-7-{D-α-/3-(3,4-Dihydro-2-formylamino-3-methyl-4-oxo-5-pyrimidyl)-ureido/-p-hydroxy-phenylacetamido}-3-/(1-methyl-tetrazol-5-yl)-thiomethyl/-ceph-3-em-4-carboxylat

Die Verbindung des Beispiels 9 läßt sich mit Pivaloyloxymethylchlorid nach der in Beispiel 3 beschriebenen Vorgehensweise in den Ester überführen.
Ausbeute 49 % der Theorie an 7-{D-α-/3-(3,4-Dihydro-2-formylamino-3-methyl-4-oxo-5-pyrimidyl)-ureido/-p-hydroxy-phenyl-acetamido}-3-/(1-methyl-tetrazol-5-yl)-thiomethyl/-ceph-3-em-4-carbonsäure-pivaloyloxymethylester
IR-Spektrum: 1770, 1740, 1670, 1620 $cm^{-1}$
NMR-Spektrum ($CDCl_3$ /$CD_3OD$) Signale bei ppm: 1,25 (s, 9H), 2,05 (s, 3H), 3,35 (s, 3H), 3,6 (m, 2H), 4,5 (m, 2H), 4,9 (d, 2H), 5,5 (s, 1H), 5,6 (d, 2H), 5,75 (q, 2H), 6,85 (d, 2H) 7,35 (d, 2H), 8,45 (s, 1H).

## Beispiel 10

a) 3.4-Dihydro-5-nitro-2-propionylamino-3-methyl-4-oxo-pyrimidin

3.6 g der entsprechenden 2-Aminoverbindung werden in 50 ml Propionsäureanhydrid 15 Minuten lang auf 140°C erhitzt. Die Lösung wird nach dem Abkühlen mit 200 ml Äther verdünnt, der ausgefallene Niederschlag abgesaugt und mit Äther gut ausgewaschen.
Ausbeute: 3.5 g (78 % der Theorie),
Schmelzpunkt: 163°C (Zersetzung)
$C_8H_{10}N_4O_4$ (226.2)

| | | | |
|---|---|---|---|
| Ber.: | C 42.45 | H 4.42 | N 24.78 |
| Gef.: | 42.50 | 4.41 | 24.65 |

b) 3,4-Dihydro-5-amino-2-propionylamino-3-methyl-4-oxo-pyrimidin

hergestellt durch Reduktion der entsprechenden Aminoverbindung in Dimethylformamid in Gegenwart von Palladium/Kohle analog Beispiel 8c.
Ausbeute: 52 % der Theorie (aus Äthanol)
$C_8H_{12}N_4O_2$ (196.2)

| | | | |
|---|---|---|---|
| Ber.: | C 48.97 | H 6.16 | N 28.56 |
| Gef.: | 48.95 | 6.17 | 28.40 |

c) D-α-/3-(3.4-Dihydro-2-propionylamino-3-methyl-4-oxo-5-pyrimidyl)-ureido/-p-hydroxybenzylpenicillin-Natrium

hergestellt analog Beispiel 8d aus 2.5 g Amoxycillin-trihydrat und dem Umsetzungsprodukt von 1.1 g Amin, 600 mg Phosgen und 570 mg Triäthylamin.
Ausbeute: 2.5 g (71 % der Theorie),
Rf-Wert: 0.64
IR-Spektrum: 1770, 1660, 1600, 1510-40 $cm^{-1}$
NMR-Spektrum ($D_2O$): Signale bei ppm 1.2 (3H), 1.5 (6H), 2.6 (2H), 3.5 (3H), 4.25 (1H), 5.3 (1H), 5.5 (2H), 7.0 (2H), 7.4 (2H), 8.45 (1H)

In analoger Weise wird hergestellt:

D-α-/3-(3.4-Dihydro-2-propionylamino-3-methyl-4-oxo-5-pyrimidyl)-ureido/-benzylpenicillin-Natrium
D-α-/3-(3.4-Dihydro-2-propionylamino-3-methyl-4-oxo-5-pyrimidyl)-ureido/-1.4-cyclohexadienyl-methylpenicillin-Natrium

Beispiel 11

a) 3.4-Dihydro-5-nitro-3-methyl-2-(3'-phenylureido)-4-oxo-pyrimidin

3.4 g der gemäß Beispiel 8a erhaltenen 2-Aminoverbindung und 3.0 g Phenylisocyanat werden bei 60°C in ca. 60 ml Dimethylformamid gelöst. Bei dieser Temperatur wird solange gerührt, bis kein Ausgangsprodukt auf dem Dünnschichtchromatogramm mehr zu erkennen ist. Es wird im Vakuum zur Trockne eingeengt, der kristalline Rückstand mit Essigester ausgerührt und abgesaugt.
Ausbeute: 3.7 g (68 % der Theorie),
Schmelzpunkt: 210°C (Zersetzung)
$C_{12}H_{11}N_5O_4$ (273,3)

| | | | |
|---|---|---|---|
| Ber.: | C 52.65 | H 4.05 | N 25.65 |
| Gef.: | 52.85 | 4.15 | 24.91 |

b) 3.4-Dihydro-5-amino-3-methyl-2-(3'-phenylureido)-4-oxo-pyrimidin

Die in Beispiel 11a erhaltene Nitroverbindung wird mit Palladium/Kohle in Dimethylformamid zur 5-Aminoverbindung hydriert.
Ausbeute: 61 % der Theorie,
Schmelzpunkt: 190°C (aus Äthanol)
$C_{12}H_{13}N_5O_2$ (259.3)

| | | | |
|---|---|---|---|
| Ber.: | C 55.60 | H 5.06 | N 27.00 |
| Gef.: | 55.61 | 5.01 | 26.41 |

Ebenso wurden erhalten:
3.4-Dihydro-5-amino-3-methyl-2-(3'-methylureido)-4-oxo-pyrimidin
3.4-Dihydro-5-amino-3-methyl-2-(3'-äthylureido)-4-oxo-pyrimidin
3.4-Dihydro-5-amino-3-methyl-2-ureido-4-oxo-pyrimidin

3,4-Dihydro-5-amino-3-methyl-2-äthoxycarbamoyl-4-oxo-pyrimidin
3.4-Dihydro-5-amino-3-methyl-2-(3'-cyclohexylureido)-4-oxo-pyrimidin

c) D-α-[3-(3.4-Dihydro-2-(3'-phenylureido)-3-methyl-4-oxo-5-pyrimidyl)-ureido]-p-hydroxybenzyl-penicillin-Natrium

hergestellt analog Beispiel 1 aus 1,9 g Amoxycillin und dem Umsetzungsprodukt des gemäß Beispiel 11b hergestellten Amins (1,2 g) mit 500 mg Phosgen und 480 mg Triäthylamin.
Ausbeute: 2.0 g Natriumsalz (62 % der Theorie),
Rf-Wert: 0,82
IR-Spektrum: 1770, 1650, 1610, 1450 $cm^{-1}$
NMR-Spektrum ($D_2O$): Signale bei ppm 1.5 (6H), 3.3 (3H), 4.2 (1H), 5.2 (1H), 5.5 (2H), 6.8 (2H), 7.3 (7H), 8.0 (1H).

In analoger Weise wird erhalten:

D-α-[3-(3.4-Dihydro-2-(3'-methylureido)-3-methyl-4-oxo-5-pyrimidyl)-ureido]-p-hydroxybenzylpenicillin-Natrium
D-α-[3-(3.4-Dihydro-2-(3'-methylureido)-3-methyl-4-oxo-5-pyrimidyl)-ureido]-benzylpenicillin-Natrium
D-α-[3-(3.4-Dihydro-2-(3'-äthylureido)-3-methyl-4-oxo-5-pyrimidyl)-ureido]-p-hydroxybenzylpencillin-Natrium
D-α-[3-(3.4-Dihydro-2-ureido-3-methyl-4-oxo-5-pyrimidyl)-ureido]-p-hydroxybenzylpenicillin-Natrium
D-α-[3-(3.4-Dihydro-2-ureido-3-methyl-4-oxo-5-pyrimidyl)-ureido]-benzylpenicillin-Natrium
D-α-[3-(3.4-Dihydro-2-ureido-3-methyl-4-oxo-5-pyrimidyl)-ureido]-1.4-cyclohexadienyl-methylpenicillin-Natrium
D-α-[3-(3.4-Dihydro-2-äthoxycarbamoylamino-3-methyl-4-oxo-5-pyrimidyl)-ureido]-p-hydroxybenzylpenicillin-Natrium

D-α-[3-(3.4-Dihydro-2-(3'-cyclohexylureido)-3-methyl-4-oxo-5-pyrimidyl)-ureido]-p-hydroxybenzylpenicillin-Natrium
D-α-[3-(3.4-Dihydro-2-(3'-cyclohexylureido)-3-methyl-4-oxo-5-pyrimidyl)-ureido]-benzylpenicillin-Natrium

## Beispiel 12

a) 3.4-Dihydro-5-nitro-3-methyl-2-dimethylamino-4-oxo-pyrimidin

6 g 3.4-Dihydro-5-nitro-3-methyl-2-methylmercapto-4-oxo-pyrimidin (siehe Beispiel 6) werden in 150 ml Methanol mit 5 g Dimethylamin bei Raumtemperatur gerührt. Nach 1 Stunde wird abgesaugt.

Ausbeute: 4.8 g (81.5 % der Theorie),
Schmelzpunkt: 215°C (Zersetzung)
$C_7H_{10}N_4O_3$ (198.2)

| | | | |
|---|---|---|---|
| Ber.: | C 42.42 | H 5.09 | N 28.27 |
| Gef.: | 42.15 | 5.10 | 28.40 |

b) 3.4-Dihydro-5-amino-3-methyl-2-dimethylamino-4-oxo-pyrimidin

4.7 g der gemäß Beispiel 12a hergestellten Nitroverbindung werden in 100 ml Dimethylformamid mit 2 g Palladium/Kohle (5 %ig) reduziert. Nach säulchenchromatographischer Aufarbeitung über Kieselgel (Methylenchlorid:Methanol 13:1) erhält man 1.4 g (26 % der Theorie) Amin.
$C_7H_{12}N_4O$ (168.2)

| | | | |
|---|---|---|---|
| Ber.: | C 49.93 | H 7.15 | N 33.44 |
| Gef.: | 49.10 | 7.31 | 32.70 |

In analoger Weise wird erhalten:

3.4-Dihydro-2.5-diamino-3-methyl-4-oxo-pyrimidin
Schmelzpunkt: 205°C (Zersetzung)

3.4-Dihydro-5-amino-2-methylamino-3-methyl-4-oxo-pyrimidin
3.4-Dihydro-5-amino-2-(N-methyl-piperazino)-3-methyl-4-oxo-pyrimidin
3.4-Dihydro-5-amino-2-äthylamino-3-methyl-4-oxo-pyrimidin

c) D-α-[3-(3.4-Dihydro-3-methyl-2-dimethylamino-4-oxo-5-pyrimidyl)-ureido]-p-hydroxybenzylpenicillin-Natrium

320 mg des in Beispiel 12 b erhaltenen Amins werden mit 200 mg Phosgen und 200 mg Triäthylamin in absolutem Tetrahydrofuran analog Beispiel 1 umgesetzt. Diese Lösung wird bei 0°C zu einer Lösung, die aus 820 mg Amoxycillintrihydrat und Triäthylamin in 80 %igem Tetrahydrofuran bereitet wurde, getropft. Die weitere Aufarbeitung erfolgt analog Beispiel 1.
Ausbeute: 440 mg (39.5 % der Theorie),
Rf-Wert: 0,59
IR-Spektrum: 1770, 1630, 1520, 1420 $cm^{-1}$
NMR-Spektrum ($D_2O$): Signale bei ppm 1.45 (3H), 1.5 (3H), 2.95 (6H), 3.4 (3H), 4.25 (1H), 5.3 (1H), 5.55 (2H), 6.9 (2H), 7.4 (2H), 8.15 (1H)

In analoger Weise wird erhalten:

D-α-[3-(3.4-Dihydro-3-methyl-2-dimethylamino-4-oxo-5-pyrimidyl)-ureido]-benzylpenicillin-Natrium
D-α-[3-(3.4-Dihydro-3-methyl-2-amino-4-oxo-5-pyrimidyl)-ureido]-p-hydroxybenzylpenicillin-Natrium
D-α-[3-(3.4-Dihydro-3-methyl-2-amino-4-oxo-5-pyrimidyl)-ureido]-benzylpenicillin-Natrium
D-α-[3-(3.4-Dihydro-3-methyl-2-amino-4-oxo-5-pyrimidyl)-ureido]-1.4-cyclohexadienylmethylpenicillin-Natrium
D-α-[3-(3.4-Dihydro-3-methyl-2-methylamino-4-oxo-5-pyrimidyl)-ureido]-p-hydroxybenzylpenicillin-Natrium

D-α-[3-(3.4-Dihydro-3-methyl-2-methylamino-4-oxo-5-pyrimidyl)-ureido]-benzylpenicillin-Natrium
D-α-[3-(3.4-Dihydro-3-methyl-2-(N-methylpiperazino)-4-oxo-5-pyrimidyl)-ureido]-p-hydroxybenzylpenicillin-Natrium
D-α-[3-(3.4-Dihydro-3-methyl-2-äthylamino-4-oxo-5-pyrimidyl)-ureido]-p-hydroxybenzylpenicillin-Natrium

Beispiel 13

Natrium-7-{D-α-[3-(3,4-Dihydro-2-amino-3-methyl-4-oxo-5-pyrimidyl)-ureido]-phenylacetamido}-3-acetoxymethyl-ceph-3-em-4-carboxylat

Herstellung analog Beispiel 2 ausgehend vom 280 mg 2,5-Diamino-3,4-dihydro-5-methyl-4-oxo-pyrimidin (0, 002 Mol) und 850 mg (0,002 Mol) Cefaloglycin-Dihydrat
Ausbeute 630 mg Natrium-Salz (48 % der Theorie)
IR-Spektrum: 1765, 1715, 1670, 1615 $cm^{-1}$
NMR-Spektrum: (DMSO + $CD_3OD$) Signale bei ppm: 2,05 (s, 3H), 3,25 (s, 3H), 3,40 (m, 2H), 4,7 (m, 2H), 4,85 (d, 2H), 5,45 (s, 1H), 5,6 (d, 2H), 7,4 (m, 5H), 8,2 (s, 1H).

Analog wurde synthetisiert:
Natrium-7-{D-α-[3-(3,4-Dihydro-2-methylamino-3-methyl-4-oxo-5-pyrimidyl)-ureido]-phenylacetamido}-3-acetoxymethyl-ceph-3-em-4-carboxylat

Beispiel 14

Natrium-7-{D-α-[3-(3,4-Dihydro-2-methylamino-3-methyl-4-oxo-5-pyrimidyl)-ureido]-p-hydroxyphenylacetamido}-3-[(1-methyl-tetrazol-5-yl)-thiomethyl]-ceph-3-em-4-carboxylat

Analog Beispiel 2 ausgehend von 460 mg 3,4-Dihydro-5-amino-3-methyl-2-methylamino-4-oxo-pyrimidin (0,003 Mol) und 1,32 g (0,003 Mol) 7-(D-α-Amino-p-hydroxy-phenylacetamido)--3-[(1-methyl-tetrazol-5-yl)-thiomethyl]-ceph-3-em-4-carbonsäure
Ausbeute 920 mg Natriumsalz (57 %)
IR-Spektrum: 1765, 1660, 1615 $cm^{-1}$
NMR-Spektrum (DMSO + $CD_3OD$) Signale bei ppm: 3,3 (s, 3H), 3,4 (s, 3H), 3,6 (m, 2H), 3,95 (s, 3H), 4,6 (durch Lösemittelpeak verdeckt), 4,85 (d, 2H), 5,45 (s, 1H), 5,60 (d, 2H), 6,8 (d, 2H), 7,35 (d, 2H), 8,15 (1H).

Analog wurde hergestellt:

Natrium-7-{D-α-[3-(3,4-Dihydro-2-methylsulfonylamino-3-methyl-4-oxo-5-pyrimidyl)-ureido]-p-hydroxyphenylacetamido}-3-[(1-methyl-tetrazol-5-yl)-thiomethyl]-ceph-3-em-4-carboxylat

Natrium-7-{D-α-[3-(3,4-Dihydro-2-ureido-3-methyl-4-oxo-5-pyrimidyl)-ureido]-p-hydroxyphenylacetamido}-3-[(1-methyl-tetrazol-5-yl)-thiomethyl]-ceph-3-em-4-carboxylat

Beispiel 15

a) 3.4-Dihydro-5-amino-3-methyl-2-morpholino-4-oxo-pyrimidin

hergestellt analog Beispiel 12a aus der entsprechenden Methylmercaptoverbindung durch Erhitzen mit Morpholin in Methanol und anschließende Reduktion der Nitrogruppe mit Palladium/Kohle in Dimethylformamid.

$C_9H_{14}N_4O_2$ (210.24)

| | | | |
|---|---|---|---|
| Ber.: | C 51.42 | H 6.71 | N 26.65 |
| Gef.: | 51.45 | 6.62 | 26.40 |

b) D-α-[3-(3.4-Dihydro-3-methyl-2-morpholino-4-oxo-5-pyrimidyl)-ureido]-p-hydroxybenzylpenicillin-Natrium

hergestellt analog Beispiel 1 ausgehend von 460 mg Amoxycillin und 250 mg des entsprechenden Amino-pyrimidins.
Ausbeute: 510 mg (69 % der Theorie),

Rf-Wert 0,65
IR-Spektrum: 1770, 1650, 1610, 1520 $cm^{-1}$
NMR-Spektrum ($D_2O$): Signale bei ppm 1,5 (6H), 3,2 (4H), 3,4 (3H), 3,9 (4H), 4,2 (1H), 5,3 (1H), 5,5 (2H), 6,8 (2H), 7,3 (2H), 8,2 (1H)

Beispiel 16

a) 3.4-Dihydro-5-amino-3-methyl-2-anilino-4-oxo-pyrimidin

4.0 g 3.4-Dihydro-5-nitro-3-methyl-2-methylmercapto-4-oxo-pyrimidin werden mit 9 g Anilin in Äthanol zum Rückfluß erhitzt. Nach 3 Stunden wird abgekühlt, das auskristallisierte Produkt abgesaugt und mit Äthanol gewaschen.
Ausbeute: 4.7 g (90 % der Theorie).

b) Die erhaltene Nitroverbindung wird anschließend mit Wasserstoff in Dimethylformamid und in Gegenwart von Palladium/Kohle reduziert.
Schmelzpunkt: 172°C
$C_{11}H_{12}N_4O$ (216.3)

| | | | |
|---|---|---|---|
| Ber.: | C 61.11 | H 5.58 | N 25.90 |
| Gef.: | 60.80 | 5.58 | 26.00 |

c) D-α-[3-(3.4-Dihydro-3-methyl-2-anilino-4-oxo-5-pyrimidyl)-ureido]-p-hydroxybenzylpenicillin-Natrium

hergestellt analog Beispiel 1 ausgehend von 1.8 g Amoxycillintrihydrat und 950 mg des entsprechenden Aminopyrimidins.
Ausbeute: 2.1 g (78 % der Theorie),
Rf-Wert: 0,81
IR-Spektrum: 1770, 1650, 1610, 1520, 1220 $cm^{-1}$
NMR-Spektrum ($D_2O$): Signale bei ppm 1.5 (6H), 3.35 (3H), 4.2 (1H), 5.2 (1H), 5.5 (2H), 6.8 (2H), 7.3 (7H), 7.95 (1H)

Beispiel 17

a) 3,4-Dihydro-5-amino-3-methyl-2-(N-methyl-N-acetylamino)-4-oxo-pyrimidin

3.0 g 3.4-Dihydro-5-nitro-3-methyl-2-methylamino-4-oxo-pyrimidin werden mit 50 ml Acetanhydrid 4 Tage lang zum Rückfluß erhitzt. Es wird in der Hitze von etwas Ausgangsprodukt abfiltriert und das Filtrat im Vakuum eingeengt. Nach Umkristallisieren des Rückstands aus Äthanol erhält man 1.8 g (48 % der Theorie).

$C_8H_{10}N_4O_4$ (226.0)

| | | | |
|---|---|---|---|
| Ber.: | C 42.20 | H 4.42 | N 24.75 |
| Gef.: | 42.00 | 4.38 | 24.65 |

Die erhaltene Nitroverbindung wird anschließend in Dimethylformamid mit Wasserstoff in Gegenwart von Palladium/Kohle reduziert.
Ausbeute: 56 % der Theorie,
Schmelzpunkt: 181°C
$C_8H_{12}N_4O_2$ (196.2)

| | | | |
|---|---|---|---|
| Ber.: | C 48.97 | H 6.16 | N 28.56 |
| Gef.: | 48.75 | 6.16 | 28.60 |

b) D-α-[3-(3.4-Dihydro-3-methyl-2-(N-methyl-N-acetylamino)-4-oxo-5-pyrimidyl)-ureido]-p-hydroxybenzylpenicillin-Natrium

hergestellt analog Beispiel 1 ausgehend von 1.1 g Amoxycillintrihydrat und 500 mg des entsprechenden Aminopyrimidins.
Ausbeute: 1.0 g (66 % der Theorie),
Rf-Wert: 0.55
IR-Spektrum: 1775, 1660, 1610, 1525 $cm^{-1}$
NMR-Spektrum ($D_2O$): Signale bei ppm 1.4 (6H), 2.2 (3H), 3.1-3.5 (6H), 4.2 (1H), 5.3 (1H), 5.5 (2H), 6.9 (2H), 7.3 (4H) 8.5 (1H).

Die nachstehenden Beispiele betreffen pharmazeutische Zubereitungen, welche Verbindungen der allgemeinen Formel I enthalten:

Beispiel A

Tabletten enthaltend D-α-[3-(3.4-Dihydro-3-methyl-2-hydroxy-4-oxo-5-pyrimidyl)-ureido]-p-hydroxybenzylpenicillin-Natrium

Ein Gemisch bestehend aus 2 kg Wirksubstanz, 5 kg Lactose, 1,8 kg Kartoffelstärke, 0,1 kg Magnesiumstearat und 0,1 kg Talk wird in üblicher Weise zu Tabletten gepreßt, derart, daß jede Tablette 200 mg Wirkstoff enthält.

Beispiel B

Dragees enthaltend D-α-[3-(3.4-Dihydro-3-methyl-2-hydroxy-4-oxo-5-pyrimidyl)-ureido]-p-hydroxybenzylpenicillin-Natrium

Analog Beispiel A werden Tabletten gepreßt, die anschließend in üblicher Weise mit einem Überzug, bestehend aus Zucker, Kartoffelstärke, Talk und Tragant überzogen werden.

Beispiel C

Kapseln enthaltend D-α-[3-(3.4-Dihydro-3-methyl-2-hydroxy-4-oxo-5-pyrimidyl)-ureido]-p-hydroxybenzylpenicillin-Natrium

5 kg Wirksubstanz werden in üblicher Weise in Hartgelatinekapseln gefüllt, derart, daß jede Kapsel 500 mg des Wirkstoffs enthält.

Beispiel D

Trockenampullen enthaltend D-α-[3-(3.4-Dihydro-3-methyl-2-hydroxy-4-oxo-5-pyrimidyl)-ureido]-p-hydroxybenzylpenicillin-Natrium

In einem aseptischen Bereich wurde 251 g Wirkstoff in 2008 ml destilliertem Wasser zur Injektion aufgelöst. Die Lösung wurde durch ein Millipore-Filter (Porengröße 0,22 um, Produkt der Millipore Corporation, Bedford, USA) filtriert. Die Lösung wurde jeweils in einer Menge von 2,0 ml in 1000 Gläschen (Kapazität 10 ml) eingegossen und es wurde lyophilisiert. Die Gläschen wurden sodann mit einem Kautschukstöpsel und einer Aluminiumkappe verschlossen. Somit wurden Gläschen (Nr. A) jeweils mit 250 mg Wirkstoff erhalten.

Eine physiologische Kochsalzlösung zur Injektion wurde in einer Menge von jeweils 2,0 ml in Ampullen abgefüllt und die Ampullen wurden verschlossen. Auf diese Weise wurden Ampullen (Nr. B) erhalten. Die physiologische Kochsalzlösung in den Ampullen (Nr. B) wurde in die Gläschen (Nr. A) gegossen, wodurch eine injizierbare Zubereitung für die intravenöse Verabreichung erhalten wurde. Destilliertes Wasser zur Injektion wurde in einer Menge von 20 ml in die Gläschen (Nr. A) gegossen und die Lösung wurde in einer 5%igen Lösung von Glucose für Injektionen (250 ml) aufgelöst. Auf diese Weise wurden Lösungen für die kontinuierliche Infusion hergestellt.

Analog sind Tabletten, Dragees, Kapseln und Ampullen erhältlich, die einen oder mehrere derübrigen Wirkstoffe der Formel I oder die physiologisch unbedenklichen Salze dieser Verbindungen enthalten.

Beispiel E

Tabletten enthaltend Natrium-7-{D-α-[3-(3,4-dihydro-2-hydroxy-3-methyl-4-oxo-5-pyrimidyl)-ureido]-p-hydroxyphenylacetamido}-3-[(1-methyl-tetrazol-5-yl)-thiomethyl]-ceph-3-em-4-carboxylat

Ein Gemisch bestehend aus 2 kg Wirksubstanz, 5 kg Lactose, 1,8 kg Kartoffelstärke, 0,1 Kg Magnesiumstearat und 0,1 kg Talk wird in üblicher Weise zu Tabletten gepreßt, derart, daß jede Tablette 200 mg Wirkstoff enthält.

Beispiel F

Trockenampullen enthaltend Natrium-7-{D-α-[3-(3,4-dihydro-2-hydroxy-3-methyl-4-oxo-5-pyrimidyl)-ureido]-p-hydroxyphenyl-acetamido}-3-[(1-methyl-tetrazol-5-yl)-thiomethyl]-ceph-3-em-4-carboxylat

Die Herstellung erfolgt wie im Beispiel D beschrieben.

**P a t e n t a n s p r ü c h e :**

1. Neue ß-Lactame der allgemeinen Formel

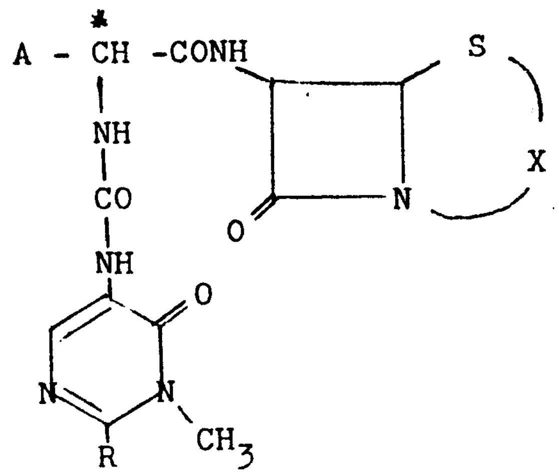

, (I)

in der

A die Phenyl-, 4-Hydroxyphenyl-, 2- oder 3-Thienyl-, 2- oder 3-Furyl-, Cyclohexyl-, Cyclohexen-1-yl-, Cyclohexa-1,4-dien-1-yl-gruppe sowie einen in 3,4-Stellung disubstituierten Phenylrest, wobei die Substituenten dieses Restes gleich oder voneinander verschieden und Chloratome, Hydroxy- oder Methoxygruppen sein können, bedeutet und R die folgenden Bedeutungen aufweist:

ein Wasserstoffatom, einen aliphatischen verzweigten oder unverzweigten gesättigten oder ungesättigten Kohlenwasserstoffrest mit 1 bis 4 Kohlenstoffatomen, die Phenyl- oder Benzylgruppe, eine Cycloalkylgruppe mit 3 bis 7 Kohlenstoffatomen, die Hydroxygruppe, eine Alkoxygruppe mit 1 bis 4 Kohlenstoffatomen, eine Alkylsulfinyl- oder Alkylsulfonylgruppe mit 1 bis 4 Kohlenstoffatomen, die Mercaptogruppe, eine Alkylmercaptogruppe mit 1 bis 4 Kohlenstoffatomen, die Aminogruppe, eine Alkyl- oder Dialkylaminogruppe mit 1 bis 8 Kohlenstoffatomen im Alkylteil, eine durch eine Hydroxygruppe substituierte Alkylaminogruppe mit 1 bis 4 Kohlenstoffatomen, eine Cycloalkylaminogruppe mit 3 bis 7 Kohlenstoffatomen, die 4-Hydroxycyclohexylaminogruppe, die Benzylaminogruppe, die Anilinogruppe, eine Morpholino-,

N-Formylpiperazino- oder N-Acetylpiperazinogruppe oder eine Gruppe der allgemeinen Formel

$$-NHCOR_1,$$

in der

$R_1$ eine Alkyl- oder Alkenylgruppe mit 1 bis 6 Kohlenstoffatomen, eine Cycloalkylgruppe mit 3 bis 7 Kohlenstoffatomen, die Benzylgruppe, die Phenylgruppe, ein Wasserstoffatom, die Trifluormethylgruppe, eine Alkoxygruppe mit 1 bis 6 Kohlenstoffatomen, die Benzyloxygruppe, eine Cycloalkoxygruppe mit 3 bis 7 Kohlenstoffatomen, die Aminogruppe, eine Alkyl- oder Dialkylaminogruppe mit 1 bis 8 Kohlenstoffatomen im Alkylteil, eine Cycloalkylaminogruppe mit 3 bis 6 Kohlenstoffatomen oder die Benzylaminogruppe darstellt; oder R bedeutet eine Gruppe der allgemeinen Formel

$$-NHSO_2R_2,$$

in der $R_2$ eine Alkylgruppe mit 1 bis 6 Kohlenstoffatomen, eine Cycloalkylgruppe mit 3 bis 7 Kohlenstoffatomen, die Benzylgruppe, die Phenyl- oder Toluylgruppe bedeutet; oder R ist eine Gruppe der Formel

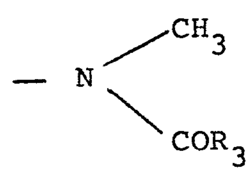
,

in der $R_3$ ein Wasserstoffatom oder eine Alkylgruppe mit 1 bis 3 Kohlenstoffatomen bedeutet,

und in der X die Gruppen:

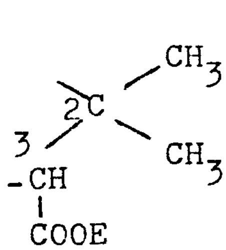
und
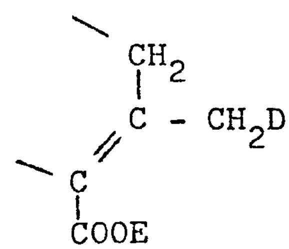

darstellt, in welchen D ein Wasserstoffatom, die Acetoxy- oder Carbamoyloxygruppe oder die Gruppe SHet bedeutet, wobei

Het die Bedeutungen einer 3-Methyl-1,2,4-thiadiazol-5-yl-, 5-Methyl-1,3,4-oxadiazol-2-yl-, 1,3,4-Thiadiazol-2-yl-, 5-Methyl-1,3,4-thiadiazol-2-yl-, Tetrazol-5-yl-, 1-Methyl-tetrazol-5-yl-, 1,2,3-Triazol-4-yl- oder 4-Methyl-oxazol-2-ylgruppe innehat und

E ein Wasserstoffatom oder eine in vitro oder in vivo leicht spaltbare Schutzgruppe darstellt,und falls E ein Wasserstoffatom bedeutet, deren physiologisch verträgliche Salze mit anorganischen oder organischen Basen.

2. Neue ß-Lactame der allgemeinen Formel I gemäß Anspruch 1, dadurch gekennzeichnet, daß
A die Phenyl-, p-Hydroxyphenyl-, 2-Furyl- oder 2-Thienylgruppe und
R ein Wasserstoffatom, die Cyclopropylgruppe, eine gegebenenfalls durch eine Alkanoylgruppe mit 1 bis 4 Kohlenstoffatomen, durch eine Trifluoracetyl-, Cyclohexanoyl-, Benzoyl-, Methylsulfonyl- oder p-Toluolsulfonylgruppe substituierte Aminogruppe, eine Monoalkylaminogruppe mit 1 bis 4 Kohlenstoffatomen, die gegebenenfalls durch eine Hydroxygruppe substituiert sein kann, die 4-Hydroxycyclohexylaminogruppe, eine gegebenenfalls in 3-Stellung durch eine Methyl-, Äthyl-, Cyclohexyl- oder Phenylgruppe substituierte Ureidogruppe, die Hydroxy-, Mercapto-, Methylmercapto-, Dimethylamino-, Äthoxycarbonylamino-, N-Acetylmethylamino-, oder Morpholinogruppe bedeuten und X, D, Het und E die in Anspruch 1 angegebenen Bedeutungen haben, und, falls E ein Wasserstoffatom bedeutet, deren physiologisch verträgliche Salze mit anorganischen oder organischen Basen.

 0006532

3. Neue ß-Lactame der allgemeinen Formel I gemäß Anspruch 1, dadurch gekennzeichnet, daß
   A die Phenyl- oder p-Hydroxyphenylgruppe und
   R ein Wasserstoffatom, die Hydroxy-, Formylamino-, Acetylamino-, Propionylamino-, Amino-, Methylamino-, Dimethylamino- oder Ureidogruppe bedeuten und X die in Anspruch 1 angegebenen Bedeutungen hat,
   worin
   D ein Wasserstoffatom, die Acetoxy-, 3-Methyl-1,2,4-thiadiazol-5-yl- oder 1-Methyl-tetrazol-5-yl-gruppe und
   E ein Wasserstoffatom oder die Pivaloyloxymethylgruppe bedeuten und, falls E ein Wasserstoffatom bedeutet, deren physiologisch verträgliche Salze mit anorganischen oder organischen Basen.

4. Neue ß-Lactame der allgemeinen Formel I gemäß den Ansprüchen 1 bis 3, dadurch gekennzeichnet, daß A, R und X die in den Ansprüchen 1 bis 3 angegebenen Bedeutungen besitzen und E als leicht abspaltbare Gruppen die Benzyl-, Diphenylmethyl-, Trityl-, t-Butyl-, die 2,2,2-Trichloräthyl-, die Trimethylsilyl-, eine Alkanoyloxyalkylgruppe mit 1 bis 5 Kohlenstoffatomen im Alkanoylrest und 1 bis 3 Kohlenstoffatomen im Alkylenrest, die Phthalidyl- oder Indanylgruppe, darstellt.

5. Neue ß-Lactame der allgemeinen Formel I gemäß Anspruch 4, dadurch gekennzeichnet, daß E die Acetoxymethyl-, Propionyloxymethyl-, 2-Acetoxyäthyl- oder Pivaloyloxymethylgruppe bedeutet.

6. Als neue Verbindungen die D-α-[3-(3,4-Dihydro-3-methyl-2-hydroxy-4-oxo-5-pyrimidyl)-ureido]-p-hydroxybenzyl-penicillansäure und die 7-{D-α-[3-(3,4-Dihydro-2-hydroxy-3-methyl-4-oxo-5-pyrimidyl)-ureido]-p-hydroxyphenylacetamido}-3-[(1-methyl-tetrazol-5-yl)-thiomethyl]-ceph-3-em-4-carbonsäure und deren

physiologisch verträgliche Salze mit anorganischen oder organischen Basen.

7. Verfahren zur Herstellung von neuen ß-Lactamen der allgemeinen Formel

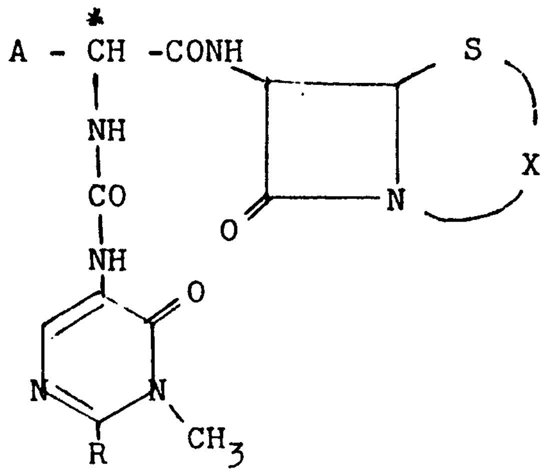

, (I)

in der

A die Phenyl-, 4-Hydroxyphenyl-, 2- oder 3-Thienyl-, 2- oder 3-Furyl-, Cyclohexyl-, Cyclohexen-1-yl-, Cyclohexa-1,4-dien-1-yl-gruppe sowie einen in 3,4-Stellung disubstituierten Phenylrest, wobei die Substituenten dieses Restes gleich oder voneinander verschieden und Chloratome, Hydroxy- oder Methoxygruppen sein können, bedeutet und R die folgenden Bedeutungen aufweist:

ein Wasserstoffatom, einen aliphatischen verzweigten oder unverzweigten gesättigten oder ungesättigten Kohlenwasserstoffrest mit 1 bis 4 Kohlenstoffatomen, die Phenyl- oder Benzylgruppe, eine Cycloalkylgruppe mit 3 bis 7 Kohlenstoffatomen, die Hydroxygruppe, eine Alkoxygruppe mit 1 bis 4 Kohlenstoffatomen, eine Alkylsulfinyl- oder Alkylsulfonylgruppe mit 1 bis 4 Kohlenstoffatomen, die Mercaptogruppe, eine Alkylmercaptogruppe mit 1 bis 4 Kohlenstoffatomen, die Aminogruppe, eine Alkyl- oder Dialkylaminogruppe mit 1 bis 8 Kohlenstoffatomen im Alkylteil, eine durch eine Hydroxygruppe substituierte Alkylaminogruppe

mit 1 bis 4 Kohlenstoffatomen, eine Cycloalkylaminogruppe mit 3 bis 7 Kohlenstoffatomen, die 4-Hydroxycyclohexylaminogruppe, die Benzylaminogruppe, die Anilinogruppe, eine Morpholino-, N-Formylpiperazino- oder N-Acetylpiperazinogruppe oder eine Gruppe der allgemeinen Formel

$$-NHCOR_1,$$

in der

$R_1$ eine Alkyl- oder Alkenylgruppe mit 1 bis 6 Kohlenstoffatomen, eine Cycloalkylgruppe mit 3 bis 7 Kohlenstoffatomen, die Benzylgruppe, die Phenylgruppe, ein Wasserstoffatom, die Trifluormethylgruppe, eine Alkoxygruppe mit 1 bis 6 Kohlenstoffatomen, die Benzyloxygruppe, eine Cycloalkoxygruppe mit 3 bis 7 Kohlenstoffatomen, die Aminogruppe, eine Alkyl- oder Dialkylaminogruppe mit 1 bis 8 Kohlenstoffatomen im Alkylteil, eine Cycloalkylaminogruppe mit 3 bis 6 Kohlenstoffatomen oder die Benzylaminogruppe darstellt;

oder eine Gruppe der allgemeinen Formel

$$-NHSO_2R_2,$$

in der

$R_2$ eine Alkylgruppe mit 1 bis 6 Kohlenstoffatomen, eine Cycloalkylgruppe mit 3 bis 7 Kohlenstoffatomen, die Benzylgruppe, die Phenyl- oder Toluylgruppe bedeutet;

oder eine Gruppe der Formel

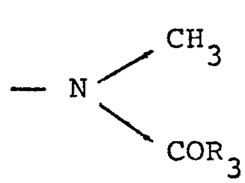
,

in der

$R_3$ ein Wasserstoffatom oder eine Alkylgruppe mit 1 bis 3 Kohlenstoffatomen bedeutet,

und in der
X die Gruppen:

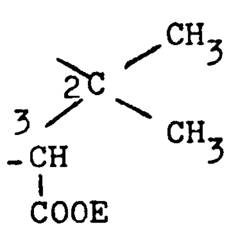

und

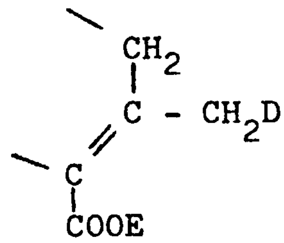

darstellt, in welchen D ein Wasserstoffatom, die Acetoxy- oder Carbamoyloxygruppe oder die Gruppe SHet bedeutet, wobei

Het die Bedeutungen einer 3-Methyl-1,2,4-thiadiazol-5-yl-, 5-Methyl-1,3,4-oxadiazol-2-yl-, 1,3,4-Thiadiazol-2-yl-, 5-Methyl-1,3,4-thiadiazol-2-yl-, Tetrazol-5-yl-, 1-Methyl-tetrazol-5-yl-, 1,2,3-Triazol-4-yl- oder 4-Methyl-oxazol-2-ylgruppe innehat
und
E ein Wasserstoffatom oder eine in vitro oder in vivo leicht spaltbare Schutzgruppe darstellt, und, falls E ein Wasserstoffatom bedeutet, von deren physiologisch verträglichen Salzen mit anorganischen oder organischen Basen, dadurch gekennzeichnet, daß

a) eine Verbindung der allgemeinen Formel

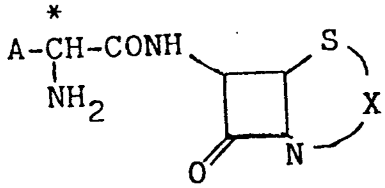

, (II)

in der A und X die oben angegebenen Bedeutungen besitzen, oder, falls E ein Wasserstoffamin ist, auch ihre Salze mit anorganischen oder organischen Basen, mit einem Pyrimidinderivat der allgemeinen Formel

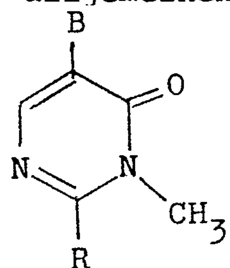

,(III)

in der R wie eingangs definiert ist und B die -N=CO oder -NH-CO-Hal-Gruppe, in der Hal ein Halogenatom bedeutet, darstellt, oder mit Gemischen solcher Pyrimidinderivaten der allgemeinen Formel III, in welchen B sowohl die eine als auch die andere Bedeutung besitzt, in einem Lösungsmittel und bei einem pH-Bereich zwischen 2,0 und 9,0 bei Temperaturen zwischen -20$^{\circ}$C und +50$^{\circ}$C, umgesetzt wird oder

b) eine Ureidocarbonsäure der allgemeinen Formel

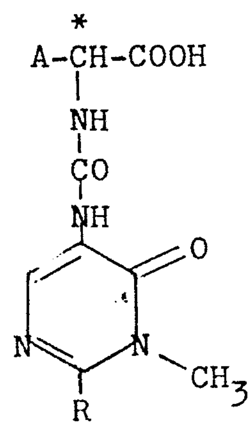

,(IV)

in der A und R die oben angegebenen Bedeutungen besitzen, oder ihre Salze oder reaktiven Derivate mit Verbindungen der allgemeinen Formel

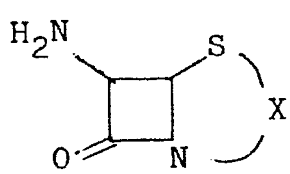

,(V)

in der X wie oben definiert ist, oder mit deren reaktiven Estern, zwischen $-40^{o}C$ und $+40^{o}C$ in Gegenwart eines Lösungsmittels und gegebenenfalls in Gegenwart einer Base umgesetzt wird und gewünschtenfalls anschließend eine erhaltene Verbindung der allgemeinen Formel I, in der in einer der möglichen Gruppen X das Symbol E für Wasserstoff steht, mittels anorganischer oder organischer Basen in ihre Salze übergeführt wird.

8. Verfahren gemäß Anspruch 7 a, dadurch gekennzeichnet, daß eine Verbindung der allgemeinen Formel II, in der E Wasserstoff bedeutet, oder eines ihrer Salze mit anorganischen oder organischen Basen mit einer Verbindung der allgemeinen Formel III,

a) in Wasser oder in mit Wasser mischbaren Lösungsmitteln in Gegenwart von Wasser in einem pH-Bereich von 6,5 bis 8,0, oder
b) in wasserfreien Lösungsmitteln oder
c) in einem Gemenge aus Wasser und mit Wasser nicht mischbaren Lösungsmitteln in einem pH-Bereich zwischen 6,5 und 8,0

ungesetzt wird oder daß eine Verbindung der allgemeinen Formel II, in der E eine Silylgruppe oder eine andere leicht abspaltbare Schutzgruppe bedeutet, mit einer Verbindung der allgemeinen Formel III in einem wasser- und hydroxylgruppenfreien bzw. aprotischen Lösungsmittel, gegebenenfalls in Gegenwart einer Base, umgesetzt wird.

9. Verfahren gemäß Anspruch 7b, dadurch gekennzeichnet, daß als reaktive Derivate der Ureidocarbonsäuren der allgemeinen Formel IV deren Säureanhydride, deren reaktive Ester oder reaktive Amide oder deren Säurehalogenide, als reaktive Ester der Verbindungen der allgemeinen Formel V der Benzhydrylester, der t-Butylester, der Trimethylsilylester oder das N,O-Bis-trimethylsilylderivat verwendet werden und die Umsetzung in Gegenwart eines tertiären Amins und/oder eines organischen Lösungsmittels und/oder eines Kondensationsmittels erfolgt und, gewünschtenfalls, das so erhaltene Produkt der allgemeinen Formel I, in der E eine abspaltbare Schutzgruppe bedeutet, anschließend in eine Verbindung der allgemeinen Formel I übergeführt wird, in der E ein Wasserstoffatom darstellt.

10. Arzneimittel gekennzeichnet durch einen Gehalt an einer oder an mehreren Verbindungen der allgemeinen Formel I gemäß den Ansprüchen 1 bis 5 neben den üblichen Träger- und/oder Hilfsstoffen.

Europäisches Patentamt

# EUROPÄISCHER RECHERCHENBERICHT

0006532

Nummer der Anmeldung

79101909.4

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | DE - A - 2 714 214 (HEYDEN)<br>+ Seiten 1 - 9 +<br>-- | 1 -10 |
| A | FR - A - 2 370 052 (MERCK)<br>+ Seiten 62 - 69 +<br>-- | 1 -10 |
| A | US - A - 4 038 271 (BREUER)<br>+ Spalten 1 - 5 +<br>-- | 1 -10 |
| A | US - A - 4 068 074 (MURAKAMI)<br>+ Spalten 1, 2 +<br>---- | 1 -10 |

KLASSIFIKATION DER ANMELDUNG (Int.Cl.²)

C 07 D 499/64
C 07 D 501/36
C 07 D 501/32
C 07 D 501/22
A 61 K 31/43
A 61 K 31/545

RECHERCHIERTE SACHGEBIETE (Int. Cl.²)

C 07 D 499/64
C 07 D 501/36
C 07 D 501/32
C 07 D 501/22
A 61 K 31/43
A 61 K 31/545

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 04-09-79 | JANISCH |